# EUROPEAN PATENT APPLICATION

(11) **EP 4 275 805 A1**
(43) Date of publication of application: **15.11.2023**
(21) Application number: 22779549.9
(22) Date of filing: 08.02.2022
(51) Int. Cl.: B21B 37/00, B21B 37/76, B21B 38/00, B21B 45/02, B21C 51/00

(54) **METHOD OF GENERATING STEEL-PLATE MATERIAL PREDICTION MODEL, MATERIAL PREDICTION METHOD, MANUFACTURING METHOD, AND MANUFACTURING FACILITY**

(30) Priority: 01.04.2021 JP 2021062617
(71) Applicant: JFE Steel Corporation, Tokyo 100-0011 (JP)
(72) Inventor: UEOKA, Satoshi, Tokyo 100-0011 (JP); TAMURA, Yuta, Tokyo 100-0011 (JP); NOJIMA, Yusuke, Tokyo 100-0011 (JP); HIRANO, Takahiro, Tokyo 100-0011 (JP); TAKEMURA, Yusaku, Tokyo 100-0011 (JP); KURIMOTO, Atsushi, Tokyo 100-0011 (JP)
(74) Representative: Grünecker Patent- und Rechtsanwälte PartG mbB
(86) International application number: PCT/JP2022/004876
(87) International publication number: WO 2022/209320

(57) **Abstract**

A method for generating a steel sheet material quality prediction model according to the present invention is a method for generating a steel sheet material quality prediction model in steel sheet cooling equipment including a water cooling device that cools a steel sheet by jetting cooling water to a heated steel sheet and a temperature measurement device that measures a surface temperature of the steel sheet in a cooling process, the method including a step of generating a material quality prediction model of the steel sheet after having passed through the cooling equipment with machine learning using a plurality of learning data in which a surface temperature information data set including actual measurement data of a surface temperature at a reference point set in advance on the steel sheet and actual measurement data of a surface temperature at a referring point set based on the reference point is set as input result data and material quality information of the steel sheet after having passed through the cooling equipment in a position corresponding to the reference point on the steel sheet corresponding to the input result data is set as output actual data.

## Description

### Field

The present invention relates to a method for generating a steel sheet material quality prediction model, a steel sheet material quality prediction method, a steel sheet manufacturing method, and steel sheet manufacturing equipment.

### Background

Mechanical properties, particularly, strength and toughness required for a steel sheet have become particularly severe in recent years. In manufacturing a steel sheet, required characteristics of the steel sheet are secured by, other than directly quenching the rolled high-temperature steel sheet, quenching in heat treatment equipment, or stopping cooling at a predetermined temperature during the direct quenching or the quenching in the heat treatment equipment. In the manufacturing, it is important to make cooling speed and a cooling stop temperature uniform over the entire width of the steel sheet such that uniform characteristics can be secured over the entire width and the entire length of the steel sheet.

Here, the quenching indicates a heat treatment method for rapidly cooling, in cooling equipment, to temperature equal to or lower than a martensitic transformation temperature (an Ms point), a steel sheet having temperature equal to or higher than an Ac3 transformation point, which is a completion temperature of austenite transformation after hot rolling and a steel sheet cooled after rolling and thereafter heated again to temperature equal to or higher than the Ac3 transformation point in a heating furnace or the like. The quenching is widely used as a particularly high-strength steel sheet manufacturing method. Quenching the rolled steel sheet without cooling and reheating is referred to as direct quenching.

On the other hand, in a cooling process, in order to generate an internal texture of ferrite, pearlite, bainite, or the like, a heat treatment process is also used in which cooling of a heated steel sheet is stopped at approximately 500°C. A technique of cooling a steel sheet after hot rolling and stopping the cooling at temperature of approximately 500°C is called accelerated cooling and is now a general heat treatment process. Further, there has also been developed a technique for controlling a composite internal texture of ferrite, pearlite, bainite, or the like by once cooling a steel sheet after hot rolling to room temperature, thereafter reheating the steel sheet, and stopping the cooling at temperature of approximately 500°C during cooling by cooling equipment for cooling the steel sheet after the reheating.

As explained above, when the internal texture of the steel sheet is controlled using the cooling process and a material of a steel sheet product is manufactured, in-plane material quality variation of the steel sheet is sometimes a problem. In general, when quality assurance concerning the material quality of the steel sheet is performed, a test piece is collected from a part of the manufactured steel sheet to acquire a mechanical test value. However, when there is the in-plane material quality variation explained above, it is likely that the test piece does not always represent the material quality of the entire surface of the steel sheet depending on a position where the test piece is collected. Therefore, there is room for improvement in the uniformity of the material quality with respect to the entire width and the entire length of the steel sheet.

In contrast, Patent Literature 1 describes a material quality assurance system in cooling equipment of a thick steel sheet manufacturing line. This system includes temperature collecting means for collecting temperature data of a steel sheet as a cooling target using temperature measurement means arranged in a plurality of positions such as an upstream position, a cooling start position, and a cooling stop position of a water cooling device, the system performing material quality assurance for a thick steel sheet based on an entire surface temperature map of the steel sheet.

On the other hand, Patent Literature 2 describes a method targeting a hot-rolled steel strip, the method being a method for disposing a thermometer in a steel strip manufacturing line including a cooling process and estimating, for each of meshes that divide a length direction, a width direction, and a thickness direction of a steel strip product, a material characteristic value for each of the meshes while referring to similarity to operation data in the past. Patent Literature 2 describes that an estimated value estimated from a temperature measurement value on the surface of the steel strip using a heat transfer model is used as temperature information inside the steel strip.

Patent Literature 3 describes a method for, targeting a process for cooling a hot-rolled steel strip, dividing a longitudinal direction of the steel strip into a plurality of regions and calculating a temperature history of the steel strip for each of the regions from cooling conditions for the regions. Patent Literature 3 describes a method for predicting a state of a micro-texture in the regions using the calculated temperature history and predicting a material quality characteristic value of the steel strip using data concerning manufacturing results in the past close to the predicted state of the micro-texture. Further, Patent Literature 3 also describes a method for, when calculating the temperature history, assuming that a temperature distribution in a thickness direction is uniform, and a method for calculating the temperature distribution in the thickness direction by solving a heat conduction equation for the thickness direction.

### Citation List

### Patent Literature

Patent Literature 1: Japanese Patent No. 5589260
Patent Literature 2: Japanese Patent No. 5924362
Patent Literature 3: JP 2012-171001 A

### Summary

### Technical Problem

However, the system described in Patent Literature 1 collects, for the entire width and the entire length of the steel sheet, temperature data of the steel sheet in cooling the steel sheet. However, even if temperature data of the entire surface of the steel sheet is collected, the material quality of the steel sheet cannot always be accurately predicted. In particular, since the temperature data collected by the temperature collecting means is measurement data concerning the surface of the steel sheet, the temperature data sometimes does not always represent a temperature history inside the steel sheet. There is room for improvement in prediction accuracy of the material quality of the steel sheet.

On the other hand, the method described in Patent Literature 2 is a method for setting a mesh defining the thickness direction of the steel strip and estimating internal temperature of the steel strip with the heat transfer model. However, the heat transfer model in the cooling process for the steel strip is usually a method for solving a first-order heat conduction equation in the thickness direction. When a heat transfer analysis in the longitudinal direction and the width direction of the steel strip is attempted to be performed, a load on a computer becomes excessive and it is difficult to use the heat transfer model as practical temperature estimating means. On the other hand, although there is no problem in practical use even if heat transfer in the longitudinal direction and the width direction of the steel strip is ignored in the in-plane center of the steel strip, heat transfer in the longitudinal direction and the width direction of the steel strip occurs in the vicinity of the tip tail end portion and the width direction end portion of the steel strip. Therefore, even if the surface temperature of the steel strip is the same, a plate thickness direction temperature distribution in the in-plane center of the steel strip and a plate thickness direction temperature distribution in the vicinity of the tip tail end portion or the width direction end portion of the steel strip are different. Therefore, in a first-order heat transfer model in the thickness direction having a surface temperature as a boundary condition, there is room for improvement in accurately estimating a temperature history inside the steel strip.

The method described in Patent Literature 3 divides the thickness direction of the steel strip, estimates the temperature history inside the steel strip by solving the heat conduction equation in the thickness direction, and predicts a state of the micros-texture in the regions based on the estimated temperature history. With the method described in Patent Literature 3, a correspondence relation with the material quality inside the steel strip is clarified in predicting not only the temperature history but also the micro-texture inside the steel strip. However, as explained above, when heat transfer occurs in the longitudinal direction and the width direction of the steel strip, there is room for improvement in the prediction accuracy of the material quality.

The present invention has been devised in view of the problems described above, and an object of the present invention is to provide a method for generating a steel sheet material quality prediction model capable of generating a steel sheet material quality prediction model for accurately predicting material quality information of a steel sheet after having passed through cooling equipment. Another object of the present invention is to provide a steel sheet material quality prediction method capable of accurately predicting material quality information of a steel sheet after having passed through cooling equipment. Further, another object of the present invention is to provide a steel sheet manufacturing method and manufacturing equipment capable of manufacturing a steel sheet excellent in material quality uniformity.

### Solution to Problem

To solve the problem and achieve the object, a method for generating a steel sheet material quality prediction model according to the present invention is the method for generating the steel sheet material quality prediction model in steel sheet cooling equipment, the cooling equipment including a water cooling device that cools a steel sheet by jetting cooling water to a heated steel sheet and a temperature measurement device that measures a surface temperature of the steel sheet in a cooling process. The method includes a step of generating a material quality prediction model of the steel sheet after having passed through the cooling equipment with machine learning using a plurality of learning data in which a surface temperature information data set including: actual measurement data of a surface temperature at a reference point set in advance on the steel sheet; and actual measurement data of a surface temperature at a referring point set based on the reference point, is set as input result data and material quality information of the steel sheet after having passed through the cooling equipment in a position corresponding to the reference point on the steel sheet corresponding to the input result data is set as output result data.

Moreover, in the method for generating a steel sheet material quality prediction model according to the present invention, at least a singularity of the referring point is set in a position in a longitudinal direction of the steel sheet and at least a singularity of the referring point is set in a position in a width direction of the steel sheet with respect to the reference point.

Moreover, in the method for generating a steel sheet material quality prediction model according to the present invention, the material quality prediction model includes, as the input result data, an attribute information parameter selected from attribute information of the steel sheet.

Moreover, in the method for generating a steel sheet material quality prediction model according to the present invention, the material quality prediction model includes, as the input result data, at least one operation result data selected from operation result data of the water cooling device.

Moreover, in the method for generating a steel sheet material quality prediction model according to the present invention, machine learning selected from a neural network, decision tree learning, random forest, and support vector regression is used as the machine learning.

Moreover, a steel sheet material quality prediction method according to the present invention is the steel sheet material quality prediction method in steel sheet cooling equipment, the cooling equipment including a water cooling device that cools the steel sheet by jetting cooling water to a heated steel sheet and a temperature measurement device that measures a surface temperature of the steel sheet in a cooling process. The method includes a step of predicting material quality information of the steel sheet after having passed through the cooling equipment by using a material quality prediction model generated by machine learning in which a surface temperature information data set including: a surface temperature at a prediction reference point set in advance on the steel sheet; and a surface temperature at a prediction referring point set based on the prediction reference point, is set as input data and material quality information of the steel sheet after having passed through the cooling equipment in a position corresponding to the prediction reference point on the steel sheet is set as output data.

Moreover, a steel sheet manufacturing method according to the present invention includes a step of performing pass/fail determination of material quality of a steel sheet after having passed through the cooling equipment using the steel sheet material quality prediction method according to the present invention.

Moreover, steel sheet manufacturing equipment according to the present invention includes: steel sheet cooling equipment including a water cooling device that cools the steel sheet by jetting cooling water to a heated steel sheet and a temperature measurement device that measures a surface temperature of the steel sheet in a cooling process; and a material quality prediction unit that outputs material quality information of the steel sheet after having passed through the cooling equipment, wherein the material quality prediction unit outputs material quality information of the steel sheet using a machine learning model in which a surface temperature information data set including: a surface temperature at a prediction reference point set in advance on the steel sheet; and a surface temperature at a prediction referring point set based on the prediction reference point, is set as input data and material quality information of the steel sheet after having passed through the cooling equipment in a position corresponding to the prediction reference point on the steel sheet is set as output data.

### Advantageous Effects of Invention

With the method for generating a steel sheet material quality prediction model according to the present invention, it is possible to generate a steel sheet material quality prediction model for accurately predicting material quality information of a steel sheet after having passed through cooling equipment. With the steel sheet material quality prediction method according to the present invention, it is possible to accurately predict material quality information of a steel sheet after having passed through cooling equipment. With the steel sheet manufacturing method and the manufacturing equipment according to the present invention, it is possible to manufacture a steel sheet excellent in material quality uniformity.

### Brief Description of Drawings

FIG. 1 is a diagram illustrating an example in which steel sheet cooling equipment according to an embodiment of the present invention is disposed in steel sheet manufacturing equipment that executes an online type heat treatment process and an offline type heat treatment process.
FIG. 2 is a diagram illustrating a configuration of the steel sheet cooling equipment according to the embodiment of the present invention when being applied to the offline type heat treatment process.
FIG. 3 is a diagram illustrating a configuration of a water cooling device illustrated in FIG. 2.
FIG. 4 is a block diagram illustrating a configuration of a control computer illustrated in FIG. 2.
FIG. 5 is a diagram for explaining a method of associating position information in a longitudinal direction of a steel sheet and the surface temperature of the steel sheet.
FIG. 6 is a diagram illustrating a measurement example of the surface temperature of the steel sheet.
FIG. 7 is a diagram illustrating an example of a reference point.
FIG. 8 is a diagram illustrating an example of a referring point.
FIG. 9 is a block diagram illustrating a configuration of a material quality prediction model generation unit according to the embodiment of the present invention.
FIG. 10 is a diagram for explaining an operation of a steel sheet material quality prediction unit according to the embodiment of the present invention.
FIG. 11 is a diagram illustrating an example of surface temperature information obtained by surface temperature measurement devices disposed in an inlet portion and an outlet portion of the cooling equipment.
FIG. 12 is a diagram illustrating a display example of yield stress and tensile strength.
FIG. 13 is a diagram illustrating a display example of a cooling device entrance side temperature, a cooling device exit side temperature, the tensile strength, and the yield stress.
FIG. 14 is a diagram illustrating an example of a relation between result values and predicted values of the tensile strength and the yield stress.
FIG. 15 is a diagram illustrating a configuration of a hot rolling line in an example.

### Description of Embodiment

A method for generating a steel sheet material quality prediction model, a steel sheet material quality prediction method, a steel sheet manufacturing method, and steel sheet cooling equipment according to an embodiment of the present invention are explained below with reference to the drawings.

### [Steel sheet manufacturing process]

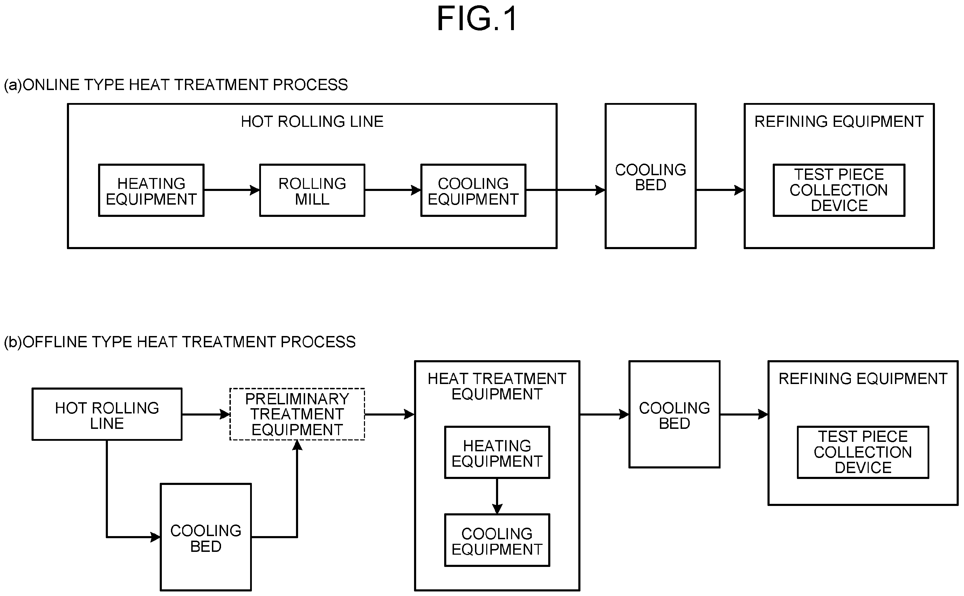

First, a steel sheet manufacturing process according to an embodiment of the present invention is explained with reference to FIGS. 1(a) and 1(b).

FIG. 1(a) is a diagram illustrating an example in which steel sheet cooling equipment according to the embodiment of the present invention is disposed in a hot rolling line for executing an online type heat treatment process. As illustrated in FIG. 1(a), in this example, first, a slab, which is a cast piece, is heated to a predetermined heating temperature by heating equipment in the hot rolling line and, thereafter, reverse rolling is performed using one or two rolling mills. Subsequently, a steel sheet rolled to a predetermined dimension by the rolling mills is conveyed from the rolling mills to the cooling equipment while keeping high temperature. In the cooling equipment, the steel sheet is cooled to a cooling stop temperature set in advance by accelerated cooling and, thereafter, the steel sheet is cooled to nearly room temperature in a cooling bed (a yard for air-cooling the steel sheet to nearly the room temperature). The steel sheet is transferred to refining equipment. In the refining equipment, the shape of the steel sheet is corrected according to necessity and defect inspection, product collection, and the like are performed. Note that the steel sheet cooled using the cooling equipment in the online type heat treatment process is sometimes subjected to a tempering process of reheating the steel sheet to temperature of 140 to 680°C and then conveyed to the cooling bed. The tempering process is different from an offline type heat treatment process explained below in that the steel sheet is not cooled using the cooling equipment after the steel sheet is reheated.

In the present embodiment, the refining equipment is provided with a test piece collection device that collects a test piece sample for a material quality inspection from the steel sheet cooled to nearly the room temperature. The test piece collection device is a device that collects a test piece sample from a position in a steel sheet surface set in advance according to a product standard, specifications, or the like. As the test piece collection device, a laser cutting machine or a shearing machine is used. The collected test piece sample is further machined into the shape of a test piece (for example, JIS No. 4 test piece in a tensile test) corresponding to an inspection item of a material quality test.

FIG. 1(b) is a diagram illustrating an example in which the steel sheet cooling equipment according to the embodiment of the present invention is disposed in heat treatment equipment that executes the offline type heat treatment process. In the offline type heat treatment process, a steel sheet rolled to a predetermined dimension in a hot rolling line is used. The hot rolling line has the same equipment configuration as the equipment configuration explained above. However, in the hot rolling line, it is not always necessary to execute the online type heat treatment process. As illustrated in FIG. 1(b), in this example, the steel sheet rolled by the hot rolling line is cooled to nearly the room temperature on the cooling bed and thereafter sent to preliminary treatment equipment. The preliminary treatment equipment is equipment for correcting the shape of the steel sheet or cutting the steel sheet into a predetermined dimension according to necessity prior to the offline type heat treatment process and sometimes performs descaling by shot blasting. However, preliminary treatment in the preliminary treatment equipment is not an essential process. Thereafter, the steel sheet is transferred to the heat treatment equipment that executes the offline type heat treatment process. The heat treatment equipment includes heating equipment. The steel sheet is heated to a predetermined temperature and thereafter cooled by the cooling equipment. The steel sheet after the heat treatment is air-cooled to nearly the room temperature in the cooling bed and thereafter conveyed to refining equipment. The refining equipment is the same as the refining equipment used in the online type heat treatment process.

### [Cooling equipment]

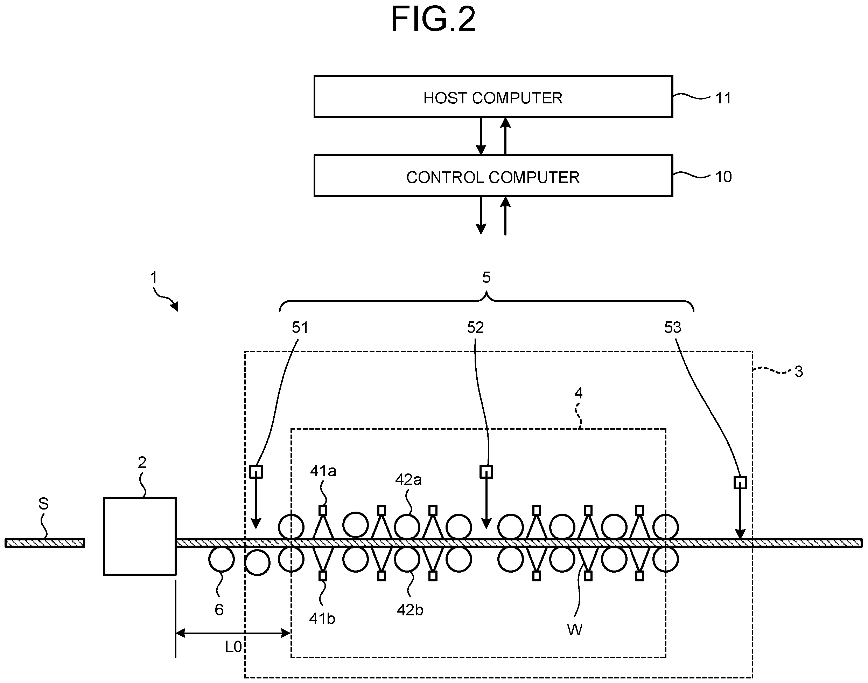

Subsequently, a configuration of the steel sheet cooling equipment according to the embodiment of the present invention is explained with reference to FIG. 2.

FIG. 2 is a diagram illustrating a configuration of the steel sheet cooling equipment according to the embodiment of the present invention when being applied to the offline type heat treatment process. As illustrated in FIG. 2, offline type heat treatment equipment 1 includes, as main components, a heating furnace 2 that heats a steel sheet S having a temperature of 100°C or lower to a predetermined temperature, cooling equipment 3 that cools the steel sheet S heated in the heating furnace 2, and a control computer 10 that controls an operation of the offline type heat treatment equipment 1 including the cooling equipment 3.

The cooling equipment 3 includes a water cooling device 4 that jets cooling water W to the steel sheet S and a temperature measurement device 5 that measures the surface temperature of the steel sheet S in a cooling process. In the present embodiment, the temperature measurement device 5 includes a temperature measurement device 51 installed on an upstream side of the water cooling device 4, a temperature measurement device 52 installed halfway in the water cooling device 4, and a temperature measurement device 53 installed on a downstream side of the water cooling device 4. However, a temperature measurement device only has to be installed in at least one position on the upstream side, halfway, and on the downstream side of the water cooling device 4. Although the water cooling device 4 illustrated in FIG. 2 is equipment including water cooling nozzles 41a and 41b and a restraint device (restraint rolls 42a and 42b), the restraint device is not essential in the present embodiment.

The heating furnace 2 is charged with the steel sheet S that has been hot-rolled to a predetermined thickness (for example, 30 mm) and width (for example, 2000 mm) by a hot rolling line present in a place different from the offline type heat treatment equipment 1 and cooled to the room temperature. The steel sheet S is heated to a predetermined temperature (for example, 910°C) in the heating furnace 2. The steel sheet S extracted from the heating furnace 2 is sent to the cooling equipment 3 while being conveyed by a plurality of table rolls 6 installed on an exit side of the heating furnace 2. In FIG. 2, the cooling equipment 3 is drawn larger than the heating furnace 2 in order to explain the configuration of the cooling equipment 3 in detail. Actually, the length of the heating furnace 2 is approximately 60 to 80 m, the equipment length of the cooling equipment 3 is approximately 20 to 25 m, and the length of the steel sheet S is longer than the equipment length of the cooling equipment 3. Therefore, at a stage when the leading end portion of the steel sheet S passes through the cooling equipment 3, a stationary portion and a tail end portion of the steel sheet S are located in the heating furnace 2.

In general, in the offline type heat treatment equipment 1, the steel sheet S is extracted from the heating furnace 2 and conveyed at substantially constant speed until the cooling by the cooling equipment 3 ends. Therefore, a cooling start temperature difference at the front tail end portion of the steel sheet S is small. That is, when a heating temperature of the steel sheet S is represented as T0, a distance from the heating furnace 2 to the water cooling device 4 of the cooling equipment 3 is represented as L0, and conveying speed of the steel sheet S is represented as V0, the leading end portion of the steel sheet S is extracted at the temperature T0 and cooled through a radiational cooling time L0/V0. In the offline type heat treatment equipment 1, since the distance L0 from the heating furnace 2 to the water cooling device 4 is short, even if the leading end portion of the steel sheet S is extracted from the heating furnace 2 and reaches an inlet of the water cooling device 4, the tail end portion of the steel sheet S is kept at the temperature T0 in the heating furnace 2. Therefore, like the leading end portion, the tail end portion of the steel sheet S is also extracted at the temperature T0 and cooled through the radiational cooling time L0/V0. Therefore, the cooling start temperature can be kept constant over the entire length of the steel sheet S. As explained above, the offline type heat treatment equipment 1 is advantageous for manufacturing a steel sheet having a small in-plane temperature deviation with respect to a thin steel sheet easily cooled by radial cooling. Therefore, there is an advantage that it is easy to secure uniformity of the material quality of the steel sheet.

In the cooling equipment used in the online type heat treatment process, unlike the cooling equipment used in the offline type heat treatment process, the heating furnace 2 is not disposed near the cooling equipment. Therefore, the stationary portion and the tail end portion of the steel sheet S are in a radially cooled state at a stage when the leading end portion of the steel sheet S passes through the cooling equipment 3. Therefore, a radial cooling time until a cooling start is longer at the tail end portion than the leading end portion of the steel sheet S. When the length of the steel sheet S is represented as L and the conveying speed of the steel sheet S is represented as V, a radial cooling time difference occurs by a time L/V between the tail end portion and the leading end portion. Even when a steel sheet temperature after rolling is uniform, the tail end portion is excessively cooled by the radial cooling time difference. Therefore, a difference occurs between cooling start temperatures of the leading end portion and the tail end portion and a temperature distribution easily occurs in the longitudinal direction in the plane of the steel sheet S. The online type heat treatment equipment has a condition under which the material quality in the plane of the steel sheet S more easily becomes nonuniform.

### [Water cooling device]

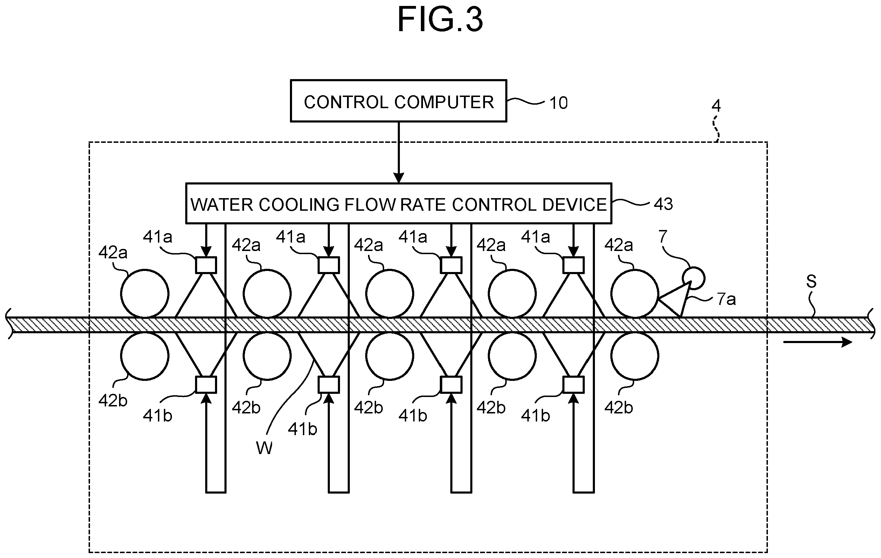

Subsequently, a configuration of the water cooling device 4 is explained with reference to FIG. 3.

FIG. 3 is a diagram illustrating the configuration of the water cooling device 4 illustrated in FIG. 2. As illustrated in FIG. 3, the water cooling device 4 includes a plurality of water cooling nozzles 41a and 41b disposed in a conveying direction of the steel sheet S to be paired in the up-down direction of the steel sheet S. The water cooling nozzle 41a jets the cooling water W downward toward the upper surface of the steel sheet S. On the other hand, the water cooling nozzle 41b jets the cooling water W upward toward the lower surface of the steel sheet S. For example, the water cooling nozzles 41a and 41b configure a pair of upper and lower water cooling nozzles. A cooling section, a unit of which is the water cooling nozzles, is referred to as cooling zone and a set of one or a plurality of cooling zones is referred to as area. In an example illustrated in FIG. 2, a cooling area (an area where water cooling is performed by the water cooling device 4) is configured from six cooling zones. In an example illustrated in FIG. 3, the cooling area is configured from four cooling zones. However, even if the number of cooling zones is other than these numbers, the effect of the present invention is not impaired. The cooling area may be configured by a plurality of cooling zones. The cooling zones may be separated by an air cooling section in which no water cooling nozzle is disposed.

As the water cooling nozzles 41a and 41b, a water cooling nozzle that includes a cooling water flow rate adjusting valve and can adjust an amount of the cooling water W jetted toward the steel sheet S is preferable. Consequently, a flow rate of the cooling water W jetted for each of the cooling zones can be adjusted. It is preferable that a water amount of the cooling water W jetted from the water cooling nozzle 41a and the water cooling nozzle 41b paired up and down toward the steel sheet S can be adjusted to different values. The water amount of the cooling water W jetted from such water cooling nozzles is controlled for each of the water cooling nozzles by a water cooling flow rate control device 43 based on a water amount setting value set by the control computer 10.

Operation parameters of the water cooling device 4 include the water amount of the cooling water W (a cooling water amount) jetted from at least the pair of water cooling nozzles 41a and 41b and speed (conveying speed) of the steel sheet S conveyed by the table roll 6. As the cooling water amount is larger, cooling speed and a temperature drop amount of the steel sheet S can be set larger. On the other hand, as the conveying speed of the steel sheet S is smaller, the temperature drop amount of the steel sheet S can be set larger. By combining these operation parameters, the cooling stop temperature and the cooling speed are controlled as cooling conditions for obtaining desired material quality.

In addition to these, the operation parameters of the water cooling device 4 include a balance of the cooling water amount for each of the cooling zones (for example, the cooling water amount is increased in the cooling zone on the upstream side and the cooling water amount is reduced in the cooling zone on the downstream side.). This is because the cooling speed can be controlled according to a temperature range of the steel sheet S. The balance of the cooling water amount for each of the cooling zones can be represented by a ratio of cooling water amounts jetted in the cooling zones. Further, the number of cooling zones in which the cooling water W is jetted may be changed. The cooling stop temperature can be controlled to different cooling stop temperatures while the cooling speed being set the same according to the number of cooling zones in use. The cooling zones in use may be specified using signs or numerical values for determining the use/non-use of the respective cooling zones. These signs or numerical values may be set as operation parameters of the water cooling device 4.

Since a heat transfer rate of the cooling water W jetted from the water cooling nozzles 41a and 41b is higher as a water amount is larger, the material quality of the steel sheet S can be controlled by adjusting the cooling speed by adjusting the cooling water amount. As the water cooling nozzles 41a and 41b, a slit-type nozzle or a flat spray nozzle that can uniformly jetting a large flow rate of the cooling water W in the width direction of the steel sheet S can be used. A porous jet nozzle or a mist nozzle may be used.

As the water cooling nozzles 41a and 41b, a nozzle that can adjust a cooling water amount for each of the water cooling nozzles may not always be used. This is because, when the water cooling device 4 includes a plurality of cooling zones, a cooling condition can be changed by changing the number of cooling zones for jetting the cooling water W.

The water cooling device 4 sometimes includes, together with the water cooling nozzles 41a and 41b, a restraint device including at least a pair of restraint rolls that restrain the steel sheet S during cooling. This is advantageous in maintaining the uniformity of cooling and securing the uniformity of the material quality of the steel sheet S by restraining strain of the steel sheet S that could occur when the steel sheet S is water-cooled. A configuration of the restraint device is explained with reference to FIG. 3.

The restraint device is disposed in the cooling area and is installed adjacent to a water cooling zone. The restraint roll 42a and the restraint roll 42b configuring the restraint device are disposed such that the axial direction of the restraint rolls is generally perpendicular to the conveying direction of the steel sheet S to restrain the steel sheet S with a pair of upper and lower rolls. Distortion due to thermal shrinkage or phase transformation occurs in the steel sheet S during the cooling by the water cooling device 4. The restraint rolls 42a and 42b are installed to restrain deformation of the steel sheet S such that the steel sheet S does not buckle because of such distortion. Consequently, it is possible to prevent, in the cooling process, the steel sheet S from being deformed, the cooling by the cooling water W from becoming non-uniform, and the material quality in the plane of the steel sheet S from becoming non-uniform. The restraint device can also include a function of a draining roll besides the function of restraining the steel sheet S. Consequently, when the temperature measurement device 5 is installed in the cooling equipment 3, it is possible to prevent water riding on the upper surface of the steel sheet S from becoming a disturbance to temperature measurement on the downstream side of the restraint device.

Note that, in the water cooling device 4 illustrated in FIG. 3, a draining purge nozzle 7 is installed on an exit side of a restraint device on the most downstream side. The draining purge nozzle 7 jets draining purge 7a at an angle in the direction of the restraint roll 42a such that the cooling water W leaking from a gap formed in a contact portion between the restraint roll 42a and the steel sheet S does not further flow to the downstream side. There is an effect of preventing a temperature deviation of the steel sheet S from enlarging because of the draining purge 7a and preventing the uniformity of the material quality of the steel sheet S from being deteriorated.

### [Control computer]

FIG. 2 is referred to again. The control computer 10 acquires, from a host computer 11, information concerning a target range of a cooling stop temperature (a target cooling stop temperature) and a target range of cooling speed (target cooling speed) necessary for obtaining desired material quality besides information concerning a heating temperature, plate thickness, plate width, weight, and the like of the steel sheet S. The control computer 10 calculates an operation condition for realizing such a condition and determines operation parameters of kinds of equipment of the water cooling device 4.

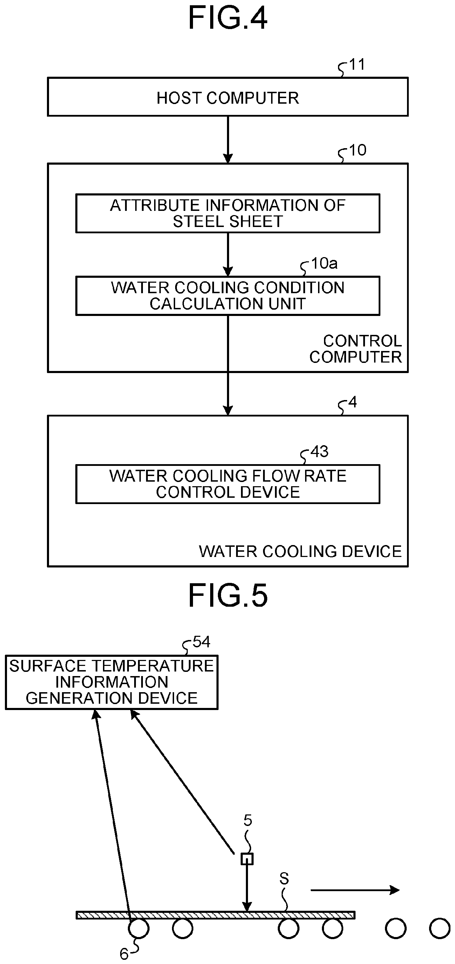

FIG. 4 is a block diagram illustrating a configuration of the control computer 10 illustrated in FIG. 2. As illustrated in FIG. 4, the control computer 10 acquires attribute information of the steel sheet S set as a target of heat treatment from the host computer 11. The attribute information of the steel sheet S includes information concerning dimensions of the steel sheet S such as sheet thickness, sheet width, sheet length, and weight of the steel sheet S, information concerning component compositions of the steel sheet S (a C content, an Si content, an Mn content, a Cr content, an Mo content, and the like of steel sheet S), and information concerning target values (yield stress, tensile strength, elongation, toughness, hardness, and the like) of mechanical characteristics of the steel sheet S after the heat treatment.

Besides the attribute information of the steel sheet S, the control computer 10 acquires information concerning a target cooling stop temperature and target cooling speed from the host computer 11. The control computer 10 performs heat transfer calculation based on an internal model in a water cooling condition calculation unit 10a, and determines operation conditions of the water cooling device 4 including a flow rate of the cooling water W of the water cooling nozzles 41a and 41b in the cooling area, a cooling zone in which the cooling water W is jetted, and conveying speed of the steel sheet S in the cooling equipment 3 to satisfy the target cooling stop temperature and the target cooling speed set as the cooling conditions.

The operation conditions of the water cooling device 4 set by the water cooling condition calculation unit 10a are sent to the water cooling flow rate control device 43. In the water cooling flow rate control device 43, commands of an operating pressure of a cooling water pump and the number of operating cooling water pumps, the number of headers provided on the upstream side of pipe systems of the water cooling nozzles 41a and 41b, an opening degree of a flow rate adjusting valve, and rotating speed of a motor that drives the table roll 6 are generated and the operation conditions of the water cooling device 4 are set. When the cooling equipment 3 includes the restraint rolls 42a and 42b and the draining purge nozzle 7 as the restraint devices, the control computer 10 also sets operation conditions thereof.

### [Temperature measurement device]

FIG. 2 is referred to again. The cooling equipment 3 of the present embodiment includes the temperature measurement device 5 that measures the surface temperature of the steel sheet S in the cooling process. The temperature measurement device 51 further on the upstream side than the water cooling device 4 is installed in a position approximately 1 to 2 m away from the inlet of the water cooling device 4. This is to measure a cooling start temperature in the heat treatment process for the steel sheet S. On the other hand, the temperature measurement device 53 further on the downstream side than the water cooling device 4 is installed in a position approximately 5 to 10 m away from the outlet of the water cooling device 4. This is to measure a cooling stop temperature in the heat treatment process for the steel sheet S. When the temperature measurement device 51 further on the upstream side than the water cooling device 4 is not installed, the inlet portion of the cooling equipment 3 coincides with the inlet portion of the water cooling device 4. On the other hand, when the temperature measurement device 53 is not installed further on the downstream side than the water cooling device 4, the outlet portion of the cooling equipment 3 coincides with the outlet portion of the water cooling device 4.

The temperature measurement device 5 used in the present embodiment has a function of measuring the surface temperature of the steel sheet S in the cooling process. The cooling process is a process of a temperature change of the steel sheet S that occurs between the inlet portion and the outlet portion of the cooling equipment 3. Therefore, the temperature measurement device 5 may be installed in any position between the inlet portion and the outlet portion of the cooling equipment 3. In the example illustrated in FIG. 2, besides the temperature measurement device 51 further on the upstream side and the temperature measurement device 53 further on the downstream side than the water cooling device 4, the temperature measurement device 52 is disposed behind three zones on the upstream side of the water cooling device 4. However, the temperature measurement device 52 between cooling zones may be installed in any position from the inlet portion to the outlet portion of the water cooling device 4 or two or more temperature measurement devices may be disposed in the water cooling device 4.

The temperature measurement device 5 may measure the surface temperature of the upper surface of the steel sheet S or may measure the surface temperature of the lower surface of the steel sheet S. As the temperature measurement device 5, either a contact type or a noncontact type may be used. In the case of the contact type, a thermocouple is preferably used. In the case of the noncontact type, a radiation thermometer is preferably used. As the radiation thermometer, besides a normal radiation thermometer that specifies the emissivity of the steel sheet S in advance and converts the emissivity from luminance data into temperature data, a two-color radiation thermometer that measures radiances at two different wavelengths and converts the measured radiances into temperatures of an object based on a ratio of the radiances may be used. Further, a more preferred embodiment is a temperature measurement device capable of measuring a temperature distribution in the width direction of the steel sheet S. Specifically, a method of arranging a plurality of radiation thermometers in a direction orthogonal to the conveying direction (the longitudinal direction) of a steel sheet or a scanning type thermometer that scans temperature measurement points in the width direction can be used. A camera-type thermometer that acquires an image of the steel sheet S and converts brightness data of the image into temperature may be used.

Note that the temperature measurement device 5 is preferably installed in a position where the temperature measurement device 5 is less easily affected by the cooling water W from the water cooling nozzles 41a and 41b. In the example illustrated in FIG. 2, the temperature measurement device 52 is partitioned by the restraint rolls 42a and 42b and is disposed in a position where the cooling water W by the water cooling nozzles 41a and 41b is not directly supplied. Consequently, it is possible to reduce disturbance with respect to the temperature measurement by the temperature measurement device 52. However, when a temperature measurement device such as an in-cooling zone steel sheet thermometer (Fountain Pyrometer) capable of measuring the surface temperature of the steel sheet S even when the cooling water W is present is used, the temperature measurement device 52 may be disposed in a position where the cooling water W is directly supplied.

In the present embodiment, the surface temperature of the steel sheet S measured by the temperature measurement device 5 is associated with position information in the plane of the steel sheet S. Concerning position information in the width direction of the steel sheet S, according to the method of arranging a plurality of radiation thermometers in fixed positions, since width direction positions where temperature data by the radiation thermometers is acquired are specified in advance, a correspondence relation between the temperature data and positions in the width direction of the steel sheet S is clear. When the temperature measurement device 5 performs scanning in the width direction using the scanning type thermometer, since a scan position in the width direction is specified by the temperature measurement device 5, the position in the width direction of the steel sheet S and temperature data to be measured are associated.

On the other hand, position information in the longitudinal direction of the steel sheet S can be associated with the temperature data acquired by the temperature measurement device 5 by specifying a conveyance distance from the leading end portion of the steel sheet S. For example, as illustrated in FIG. 5, when the temperature measurement device 5 is disposed, the leading end portion of the steel sheet S reaching the temperature measurement device 5 can be determined by the measured temperature data increasing stepwise. With a signal indicating that the leading end portion of the steel sheet S has reached the temperature measurement device 5 as a trigger, the subsequent conveyance distance is calculated from the rotation speed and the diameter of the table roll 6 that conveys the steel sheet S. The conveyance distance is position information in the longitudinal direction from the leading end portion of the steel sheet S. The measurement value of the surface temperature of the steel sheet S measured by the temperature measurement device 5 and the position information in the longitudinal direction are sent to, for example, a surface temperature information generation device 54. The measurement value of the surface temperature of the steel sheet S is associated with the position information in the plane of the steel sheet. Note that a method of associating the surface temperature of the steel sheet S and the position information in the plane of the steel sheet S is not limited to such a method. When the entire surface of the steel sheet S is measured by a camera-type two-dimensional thermometer, temperature and position information on the entire surface of the steel sheet S are acquired. Therefore, the surface temperature of the steel sheet S and the position information in the plane of the steel sheet S are simultaneously acquired.

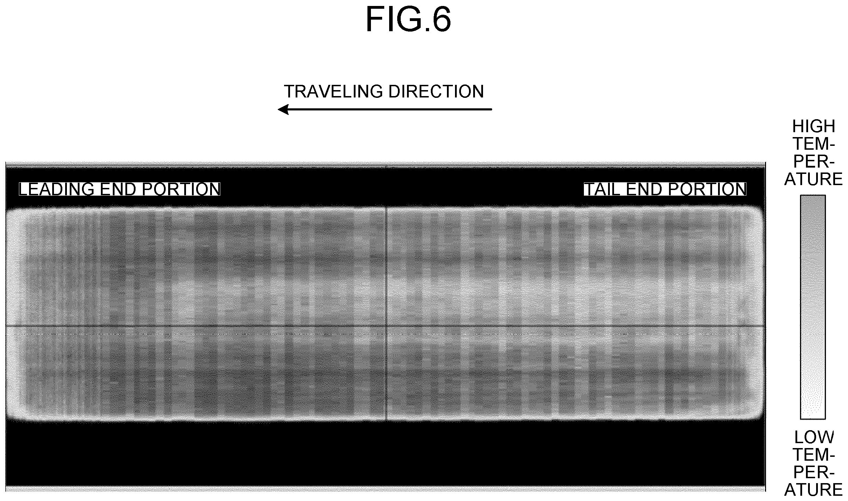

FIG. 6 illustrates a measurement example of the surface temperature of the steel sheet S. In the surface temperature information in the plane of the steel sheet S, measured temperature is associated with the position information in the plane of the steel sheet S by setting a two-dimensional coordinate system on the surface of the steel sheet S. The surface temperature information associated with the position information is sent to the control computer 10 or the host computer 11 and stored in a storage device of at least one of the control computer 10 and the host computer 11.

### [Reference point and referring point]

In the method for generating a steel sheet material quality prediction model in the present embodiment, actual measurement data of a surface temperature at a reference point set in advance on the steel sheet S and actual measurement data of a surface temperature at a referring point set based on the reference point are acquired using the temperature measurement device 5 installed in the cooling equipment 3. The acquired actual measurement data of the surface temperatures at the reference point and the referring point is hereinafter referred to as a surface temperature information data set.

The reference point in the present embodiment is a point optionally set in the plane of the steel sheet S to be cooled and refers to a point, the position of which in the plane of the steel sheet S is specified. That is, the reference point is a point, the position of which is specified by the distance from the leading end portion or the tail end portion in the plane of the steel sheet S and the distance from one width direction end portion or the other width direction end portion of the steel sheet S.

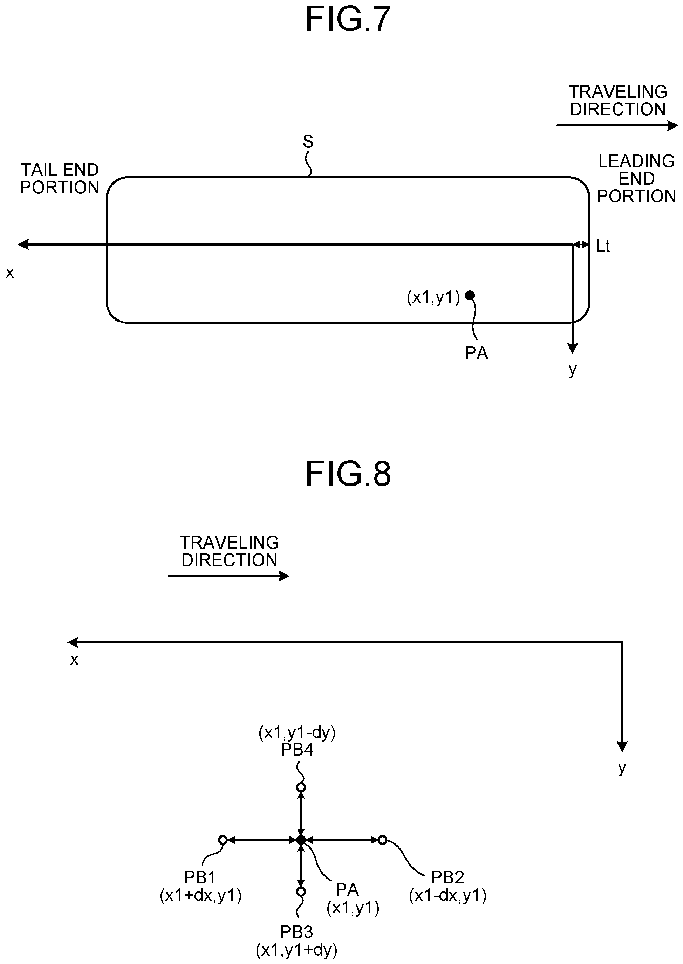

FIG. 7 illustrates an example of the reference point. As illustrated in FIG. 7, when a direction from the leading end portion to the tail end portion of the steel sheet S with respect to a traveling direction of the steel sheet S is represented as an x axis and a direction on the right hand side in the traveling direction of the steel sheet S is represented as a y axis, a reference point PA is a point, the position of which is specified by a coordinate (x1, y1). A plurality of reference points may be set for one steel sheet S. In the example illustrated in FIG. 7, since the planar shape of the steel sheet S is not always a rectangular shape, a coordinate system is set with the origin set in a position separated by a preset distance Lt (for example, Lt can be set to approximately 0.05 to 0.3 m) in the direction from the width direction center of the most leading end portion to the tail end portion of the steel sheet S to exclude a region where lack of width occurs in the most leading end portion of the steel sheet S. Since the region where the lack of width occurs at the most leading portion of the steel sheet S is not a steel sheet product, the region may be excluded from a target region of material quality prediction for the steel sheet S in the present embodiment.

On the other hand, the referring point is a point set based on the reference point and refers to a point, a positional relation of which with the reference point in the steel sheet surface is specified. As the referring point, a plurality of points may be set for one reference point. FIG. 8 illustrates an example of the referring point. referring points PB1 and PB2 are set on the tail end side and the leading end side separated from the reference point PA specified by the coordinates (x1, yl) by a distance dx in the traveling direction. In addition, referring points PB3 and PB4 are set on the right hand side and the left hand side in the traveling direction separated by a distance dy in the width direction. At least one referring point only has to be set for one reference point and may be set either in the traveling direction or in the width direction of the steel sheet S. The referring point may be set in a position having a fixed angle with respect to the traveling direction of the steel sheet S and does not always need to be set in the traveling direction and the width direction of the steel sheet S. However, it is preferable to set the referring point in both directions of the traveling direction and the width direction of the steel sheet S with respect to the reference point and it is more preferable to set two or more referring points in the directions.

The distance between the referring point and the reference point can be optionally set in a range of 0.1 to 200 mm. The distance is preferably 1 to 50 mm and more preferably 5 to 20 mm. The distance between the referring point and the reference point may be changed according to the sheet thickness of the steel sheet S. For example, the distance between the referring point and the reference point may be set from a range of 0.5 H to 3.0 H with respect to sheet thickness H of the steel sheet S. As explained below, in the present embodiment, behavior of heat transfer inside the steel sheet is indirectly specified by the difference between surface temperatures measured in the referring point and the reference point. Therefore, if the distance between the referring point and the reference point is too short, the difference between the surface temperatures of the referring point and the reference point is less easily detected. On the other hand, when the distance between the referring point and the reference point is too long, a material quality distribution due to a position in the steel sheet surface is less easily specified. Since the heat transfer between the referring point and the reference point depends on time, the distance between the referring point and the reference point may be set according to target cooling speed. For example, when the target cooling speed is high, the distance between the referring point and the reference point may be reduced and, when the target cooling rate is low, the distance between the referring point and the reference point may be increased.

Note that, in the present embodiment, as explained above, the surface temperature of the steel sheet S is associated with the in-plane position information by the temperature measurement device 5. For this reason, surface temperature data of the referring point and the reference point can be specified based on the position information of the referring point and the reference point. Consequently, a surface temperature information data set can be configured.

### [Material quality Information of the steel sheet]

In the method for generating a steel sheet material quality prediction model according to the embodiment of the present invention, the surface temperature information data set is included in input result data. Material quality information of the steel sheet S after having passed through the cooling equipment 3 in a position corresponding to the reference point on the steel sheet S corresponding to the input result data is set as output result data.

Result data of the material quality information of the steel sheet S is acquired from the steel sheet S after having passed through the cooling equipment 3 and can be obtained from the steel sheet S after being cooled to nearly the room temperature. Specifically, in the example illustrated in FIGS. 1(a) and 1(b), material quality information can be acquired from the steel sheet S at a stage of being conveyed to the cooling bed or the refining equipment after having passed through the cooling equipment 3. From the steel sheet S cooled to nearly the room temperature, a test piece sample for acquiring material quality information is collected by a test piece collection device of the refining equipment. In quality assurance of the steel sheet S to be a product, test items for inspecting material quality of the steel sheet S and a collection position of the test piece sample are set in advance. The sampling position of the test piece sample is often set and specified in advance according to a size, a standard, and specifications of the steel sheet S. Therefore, the material quality information of the steel sheet S is acquired in association with the position information of the steel sheet S. Note that, in the online type heat treatment process illustrated in FIG. 1(a), when the steel sheet cooled by the cooling equipment is conveyed to the cooling bed through the tempering process for performing reheating, result data of the material quality information of the steel sheet S is acquired from the steel sheet S after being subjected to the tempering treatment. This is because the material quality information of the steel sheet S is changed by the tempering treatment, although the steel sheet S is not cooled using the cooling equipment in the tempering process.

In the present embodiment, the material quality information of the steel sheet S refers to information concerning mechanical characteristics of the steel sheet S manufactured through the heat treatment process. The information concerning the mechanical characteristics refers to information obtained from tests usually performed to specify the mechanical characteristics of the steel sheet S such as a tensile test, a compression test, a bending test, a Charpy impact test, a CTOD test, a DWTT test, and a fatigue test. In the case of the tensile test, tensile strength, yield strength, and elongation (permanent elongation, breaking elongation, total elongation, and the like) can be used as information obtained from a test based on a standard such as JIS or ISO. Since an upper yield point, a lower yield point, 0.2% yield strength, and aperture are also information obtained from the tensile test, these are material quality information of the steel sheet S. In the case of the Charpy impact test, a V-notch test piece is collected from a test piece sample and absorbed energy and a brittle fracture surface ratio at the time of fracture by a pendulum hammer acquired for each test temperature can be set as the material quality information of the steel sheet S. In the CTOD test, a crack tip opening amount (a limit CTOD value) at which unstable fracture occurs, which is acquired for each test temperature, can be set as the material quality information of the steel sheet S. In general fatigue fracture, the number of times of repetition of fracture acquired for each set stress amplitude or a value of a fatigue limit may be set as the material quality information of the steel sheet S.

When test piece samples are acquired from a plurality of positions of the steel sheet S, material quality information is obtained for each of test piece sample collection positions according to a test piece sample collection position in the plane of the steel sheet S. In this case, it is possible to obtain the material quality information of the steel sheet S in which the respective test piece sample collection positions are set as reference points. When a plurality of test piece samples are collected from the same part of the steel sheet S, a plurality of types of material quality information can be obtained using respective test pieces. In this case, a plurality of pieces of acquired material quality information can be set as the material quality information of the steel sheet S as a set of data sets.

### [Material quality prediction model generation unit]

A steel sheet material quality prediction device according to the embodiment of the present invention includes a material quality prediction model generation unit. In the cooling equipment 3, the material quality prediction model generation unit generates a material quality prediction model of the steel sheet S after having passed through the cooling equipment 3 through machine learning using a plurality of learning data in which a surface temperature information data set including actual measurement data of a surface temperature at a reference point set in advance on the steel sheet S and actual measurement data of a surface temperature at a referring point set based on the reference point is set as input result data and material quality information of the steel sheet S after having passed through the cooling equipment 3 in a position corresponding to the reference point on the steel sheet S corresponding to the input result data is set as output result data. The material quality information of the steel sheet S after having passed through the cooling equipment 3, which is the output result data, is not always limited to the material quality information after being cooled to the room temperature after the steel sheet S has passed through the cooling equipment 3. However, when the tempering process for reheating the steel sheet S after being passed through the cooling equipment 3 is executed, the material quality information of the steel sheet S after completion of the tempering process is set as the output result data.

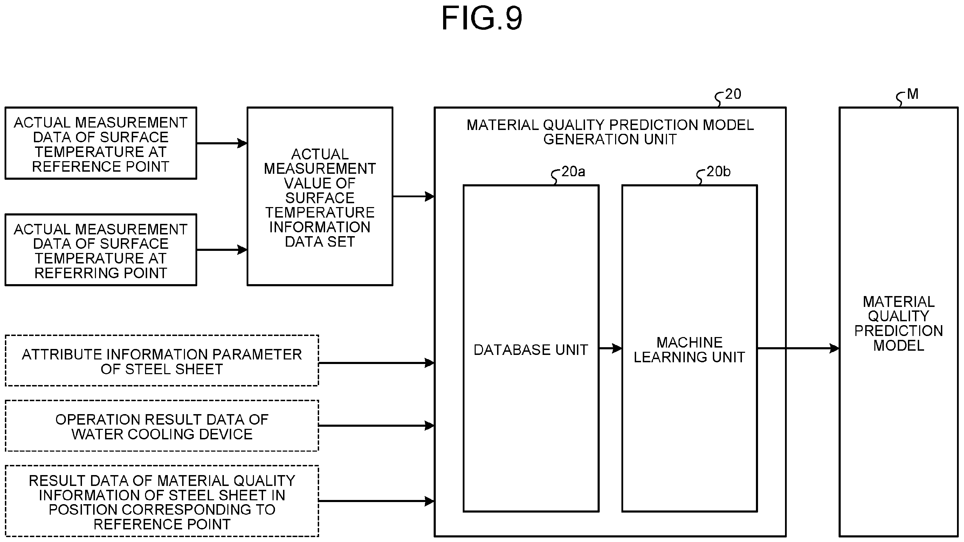

FIG. 9 illustrates a configuration of the material quality prediction model generation unit according to the embodiment of the present invention. As illustrated in FIG. 9, the material quality prediction model generation unit 20 according to the embodiment of the present invention includes a database unit 20a and a machine learning unit 20b. The database unit 20a acquires actual measurement data of a surface temperature at a reference point on the steel sheet S and actual measurement data of a surface temperature at a referring point associated with the reference point as actual values of the surface temperature information data set and acquires material quality information of the steel sheet S after being passed through the cooling equipment 3 in a position corresponding to the reference point.

The surface temperature information data set accumulated in the database unit 20a is, as a reference point on the steel sheet S, surface temperature information data corresponding to a position where material quality information is acquired. Therefore, when a plurality of positions where the material quality information of the steel sheet S is acquired are present on one steel sheet S, surface temperature information data sets and material quality information corresponding to reference points of the positions are accumulated in the database unit 20a in association with each other. That is, the number of data sets accumulated in the database unit 20a is the same as the number of reference points from one steel sheet S.

In the result data of the material quality information of the steel sheet S, the position information in the plane of the steel sheet is specified as the position where the test piece sample is collected as explained above. However, since the test piece sample for acquiring the material quality information of the steel sheet S has a fixed size according to a test method, the test piece sample does not strictly coincide with the position of the reference point configuring the surface temperature information data set. However, since it is common technical knowledge that a normal idea of quality assurance is evaluated on the premise that material quality is uniform in the test piece sample, even in the present embodiment, the position where the result data of the material quality information of the steel sheet is acquired and the reference point configuring the surface temperature information data set do not need to strictly coincide. Therefore, in the "position corresponding to the reference point on the steel sheet" explained above, a coordinate specified in the plane of the steel sheet S of the reference point only has to be included in the range of the test piece sample collected by the test piece collection device. Preferably, the center of the position where the test piece for acquiring the material quality information is collected is within 200 mm from the reference point configuring the surface temperature information data set.

The database unit 20a may accumulate a parameter (an attribute information parameter) concerning the attribute information of the steel sheet S as input result data. As explained above, the attribute information of the steel sheet S is the information concerning the dimensions of the steel sheet S such as the sheet thickness, the sheet width, the sheet length, and the weight of the steel sheet S, the information concerning the component composition of the steel sheet S (the C content, the Si content, the Mn content, the Cr content, and the Mo content of the steel sheet S) and the information concerning the target value of the mechanical characteristic of the steel sheet S after the heat treatment (the yield stress, the tensile strength, the elongation, the toughness, the hardness, and the like). The information concerning the component compositions of the steel sheet S may include contents of Nb, Ni, V, W, Sn, and Cu besides the contents of C, Si, Mn, Cr, and Mo. However, since one set of attribute information of the steel sheet S is associated with one steel sheet S, even when a plurality of reference points are set for one steel sheet S and actual measurement values of a plurality of surface temperature information data sets are obtained, the attribute information parameter of the same steel sheet S is associated with the surface temperature information data sets. The attribute information parameter of the steel sheet S is used for generation of the material quality prediction model because this is advantageous in that a highly accurate material quality prediction model can be generated even when the attribute information of the steel sheet S greatly changes. However, when the material quality prediction model is generated for the steel sheet S of the same steel type (a management range of component compositions is common), it is unnecessary to include the attribute information parameter of the steel sheet S in the input to the material quality prediction model. This is because, when the management range of the component compositions is common, even if actual values of the component compositions of the steel sheet S fluctuate, a large variation does not occur in the material quality information of the steel sheet S.

In the database unit 20a, result data (operation result data) of an operation result parameter of the water cooling device 4 may be accumulated. As explained above, as the operation result parameter of the water cooling device 4, it is possible to use setting values or actual measurement values of operation parameter that affect a cooling state of the steel sheet S such as a water amount of the cooling water W (cooling water amount) jetted from the water cooling nozzles 41a and 41b, speed (conveying speed) of the steel sheet S by the table roll 6, information regarding balance of the cooling water amount for each of the cooling zones, and the number of cooling zones in which the cooling water W is jetted. This is because a cooling state of the steel sheet S affects the material quality of the steel sheet S after the cooling is completed. An upper-lower water amount ratio of the cooling water W jetted from the water cooling nozzles 41a and 41b may be set as an operation result parameter of the water cooling device 4. This is because a warp occurs in the steel sheet S depending on the upper-lower water amount ratio of the cooling water W and affects the cooling state of the steel sheet S.

When an operation condition concerning the cooling water amount is used as the operating result parameter of the water cooling device 4, a cooling water amount for each the water cooling nozzles can be set as the operation result data of the water cooling device 4 by allocating identification numbers to the water cooling zones and the upper and lower water cooling nozzles 41a and 41b. However, the sum of cooling water amounts in the water cooling zones or the sum of cooling water amounts in a plurality of water cooling zones optionally selected from the water cooling zones may be set as the operation result data of the water cooling device 4. In particular, in a plurality of cooling zones on the front stage side (the upstream side) of the cooling area, since a temperature change of the steel sheet S is large and the influence on the material quality of the steel sheet S is large, the sum of cooling water amounts in two to three cooling zones on the front stage side of the cooling area may be used.

When a flowmeter is installed in a unit of a water cooling zone, a water cooling nozzle, a water cooling header on the upstream side of a piping system of the water cooling nozzle, or the like, result data acquired by the flowmeter may be used as the operation result data of the water cooling device 4. However, the set value of the cooling water amount set in the water cooling condition calculation unit 10a may also be used. This is because, if a set value and a result value of the water cooling nozzle are compared in advance, it is considered that a result cooling water amount less often greatly deviates from the set value.

Further, cooling speed of the steel sheet S and conveying speed of the steel sheet S in the cooling equipment 3 may be used as the operation result data of the water cooling device 4. This is because a temperature gradient that occurs in the longitudinal direction of the steel sheet S is changed by the cooling speed of the steel sheet S and the shape of the steel sheet S due to a gradient of thermal strain in the longitudinal direction is changed to thereby affect a temperature history of the steel sheet S and affect the material quality of the steel sheet S after having passed through the cooling equipment.

Besides, the operation result data of the water cooling device 4 may include the cooling stop temperature of the steel sheet S. This is because, when a cooling stop temperature is low, since the steel sheet S enters a cooling region of nuclear boiling and is in a condition under which a temperature deviation easily occurs, uniformity of the material quality in the plane of the steel sheet S is sometimes deteriorated.

From the above, when the operation result data of the water cooling device 4 is used as the input result data of the steel sheet material quality prediction model, the operation result data preferably includes at least one of the cooling water amount, the upper-lower water amount ratio of the cooling water W, the cooling speed of the steel sheet S, and the conveying speed of the steel sheet S in the cooling equipment 3 and more preferably includes a plurality of pieces of operation result data among these. This is because it is advantageous for predicting a distribution of the material quality in the plane of the steel sheet S caused by a plurality of causes.

When the operation result data of the water cooling device 4 explained above is used, an average value or the like of the operation result data in the plane of the steel sheet S can be calculated and the calculated value can be used for the operation result data of the water cooling device 4 as a representative value. At this time, when a plurality of surface temperature information data sets are acquired for one steel sheet S, the same representative value is associated with the respective surface temperature information data sets as the operation result data of the water cooling device 4. On the other hand, when the cooling water amount, the number of cooling zones in use, and the like change according to the position in the longitudinal direction of the steel sheet in the cooling process for the steel sheet S, the cooling water amount, the number of cooling zones, and the like used when the reference point passes through the cooling equipment 3 may be accumulated in the database unit 20a as the operation result data of the water cooling device 4.

Input result data of a material quality prediction model M is not limited to the input result data explained above and may include operation result data of the heating furnace 2 such as result values or set values of temperature results, residence times, and the like in zones such as a heating zone and a soaking zone in the heating furnace 2 of the offline type heat treatment equipment 1. This is because surface roughness and a state of an oxide of the steel sheet S affect the wettability of the cooling water W and indirectly affects the material quality of the steel sheet S because a temperature distribution in the plane of the steel sheet S during cooling changes. Further, when a restraint device is disposed in the cooling area, operation result data of the restraint device such as a restraint force of the steel sheet S by the restraint rolls 42a and 42b configuring the restraint device, a setting value or an actual measurement value of a depression position may be included in the input result data of the material quality prediction model M. This is because the shape of the steel sheet S in the cooling process sometimes changes because of these factors, which affects the uniformity of the material quality in the plane of the steel sheet S after having passed through the cooling equipment 3. When the draining purge nozzle 7 is provided on an exit side of the restraint device on the most downstream side of the water cooling device 4, operation result data of the draining purge nozzle 7 such as a purge pressure and a gas jetting amount from the draining purge nozzle 7 may be included in the input result data of the material quality prediction model M. This is because, if an operation condition of the draining purge nozzle 7 is not appropriate, a temperature deviation of the steel sheet S expands and the uniformity of the material quality of the steel sheet S is deteriorated.

The material quality prediction model generation unit 20 may be present on the inside of the control computer 10 or may be incorporated in a host computer 11 that gives a manufacturing instruction to the control computer 10. The material quality prediction model generation unit 20 may be configured by separate hardware capable of communicating with the control computer 10 and the host computer 11. The material quality prediction model generation unit 20 can also be included in a material quality determination unit that determines acceptance or rejection of material quality explained below.

As explained above, the result data of the surface temperature information data set corresponding to the reference point set in advance on the steel sheet S and associated with the test piece sample collection position, the material quality information in the position corresponding to the reference point of the steel sheet S after having passed through the cooling equipment, the operation result data such as the attribute information of the steel sheet S acquired according to necessity, and the operation result data of the water cooling device 4 configure one set of data sets for each of reference points set in advance and are accumulated in the storage device of the database unit 20a. The one set of data sets configured for each of the reference points may include one or two or more operation result data selected from the operation result data of the heating furnace 2, the operation result data of the restraint device, and the operation result data of the draining purge nozzle 7. Since the number of test piece sample collection positions set in the plane of the steel sheet S is usually set to approximately one to ten, in the present embodiment, approximately one to ten data sets for one steel sheet are accumulated in the storage device of the database unit 20a.

In the database unit 20a, fifty or more data sets are accumulated for each section of the same standard, the same steel type, and the same size. The number of data sets is preferably 100 or more and more preferably 500 or more. When the steel sheet S different in any one of a standard, a steel type, and a size is included, 2000 or more data sets are preferably accumulated. When the standard or the steel type of the steel sheet S is different, the influence of a component composition on the material quality of the steel sheet S after the heat treatment increases. Therefore, an attribute information parameter of the steel sheet S is preferably included in the data set accumulated in the database unit 20a.

Screening is sometimes performed on the data accumulated in the database unit 20a according to necessity. Data indicating an abnormal value may be removed. This is because highly reliable data is accumulated and the prediction accuracy of the material quality is improved. A fixed number of data sets may be set as an upper limit of the data sets accumulated in the database unit 20a. The data sets accumulated in the database unit 20a may be updated as appropriate within the upper limit.

The machine learning unit 20b generates, using the data set accumulated in the database unit 20a, the material quality prediction model M of the steel sheet S after having passed through the cooling equipment 3 through machine learning using a plurality of learning data in which the surface temperature information data set corresponding to the reference point set in advance on the steel sheet S is set as the input result data and the material quality information of the steel sheet after having passed through the cooling equipment 3 in the position corresponding to the reference point on the steel sheet S corresponding to these input result data is set as the output result data. The machine learning may be performed by including, in the input result data, one or two or more operation result data selected from the attribute information parameter of the steel sheet S, the operation result data of the water cooling device 4, the operation result data of the restraint device, the operation result data of the heating furnace 2, and the operation result data of the draining purge nozzle 15 accumulated in the database unit 18a according to necessity.

The machine learning model for generating the material quality prediction model M may be any machine learning model if prediction accuracy of material quality information sufficient for practical use can be obtained. For example, a neural network (including deep learning and a convolutional neural networks), decision tree learning, random forest, support vector regression, and the like, which are generally used, only has to be used. An ensemble model obtained by combining a plurality of models may be used.

Further, as the material quality prediction model M, not only the material quality information of the steel sheet S is output as a numerical value but also a machine learning model in which data obtained by determining whether the material quality information is within a predetermined allowable range of material quality information and binarizing a result of the determination as pass and fail is set as output result data may be used. At that time, a classification model such as K-nearest neighbor algorithm or logistic regression can be used.

Note that the material quality prediction model M may be updated to a new model by relearning, for example, every month or every year. This is because, as data stored in the database unit 20a increases, more accurate material quality prediction is possible and the material quality prediction model M reflecting a change with time of the operation condition can be generated by updating the material quality prediction model M based on the latest data.

Here, a reason why actual measurement data of a surface temperature at a reference point set in advance on the steel sheet S and actual measurement data of a surface temperature at a referring point set based on the reference point are used for input to the material quality prediction model M is explained.

In the present embodiment, a steel sheet set as a target of material quality prediction usually has sheet thickness of 3 to 100 mm, sheet width of 1000 to 4000 mm, and length of 4000 to 20000 mm. For such a steel sheet, in the conventional material quality prediction model, a temperature distribution inside the steel sheet is estimated by heat transfer calculation or the like based on a measurement result of the surface temperature of the steel sheet in a cooling process and the material quality of the steel sheet is predicted from information concerning a correspondence relation between a thermal history inside the steel sheet and the material quality after cooling, which is known in advance. In this case, heat transfer calculation for estimating a temperature distribution inside the steel sheet is based on a first-order heat conduction equation in the sheet thickness direction. That is, information concerning a measured value concerning the surface temperature of the steel sheet is only used for temperature estimation on the inside in a direction perpendicular to the surface of the steel sheet from a measurement position. On the other hand, when the sheet thickness of the steel sheet is 5 mm or more, not only heat transfer behavior in the sheet thickness direction but also influence on an internal temperature of the steel sheet by in-plane heat transfer cannot be ignored in some cases. In particular, it is necessary to consider heat transfer in the in-plane direction inside the steel sheet in a region where influence by heat transfer can occur from the end surface of the steel sheet such as the leading end portion, the tail end portion, and the width direction end portion of the steel sheet.

In contrast, in the present embodiment, not only actual measurement data of a surface temperature at a reference point set in advance on the steel sheet but also actual measurement data of a surface temperature at a referring point set based on the reference point are used. Consequently, behavior of in-plane heat transfer inside the steel sheet is reflected on the difference between the surface temperature at the reference point and the surface temperature at the referring point. That is, when the surface temperature at the reference point is higher than the surface temperature at the referring point, it is estimated that heat transfer inside the steel sheet has occurred from immediately below the reference point to immediately below the referring point. Compared with the conventional method in which only the surface temperature information at the reference point is used, a material quality prediction model reflecting information concerning the in-plane heat transfer inside the steel sheet can be obtained by combining the information concerning the surface temperature at the referring point. In the present embodiment, information concerning the heat transfer behavior in the sheet thickness direction of the steel sheet is acquired by the surface temperature at the reference point and information concerning the heat transfer behavior in the in-plane direction inside the steel sheet is acquired by the relation between the surface temperatures at the reference point and the referring point. Therefore, the information concerning the surface temperature is information for specifying heat transfer behavior on the inside of the steel sheet. It is known that the heat transfer behavior on the inside in the heat treatment process for the steel sheet greatly affects the material quality of the steel sheet after the heat treatment. By a machine learning method using the surface temperature information data set as input and using the material quality information of the steel sheet as output, a correlation between the surface temperature information data set and the material quality information of the steel sheet can be quantitatively reflected on a material quality prediction model and a highly accurate steel sheet material quality prediction model can be generated.

Further, by setting a plurality of referring points with respect to a preset reference point, it is possible to accurately reflect behavior concerning heat transfer inside the steel sheet. By setting at least one referring point in a position in the longitudinal direction of the steel sheet and at least one referring point in a position in the width direction of the steel sheet, it is possible to reflect information concerning a direction of heat transfer occurring on the inside immediately below the reference point. For example, in the referring point illustrated in FIG. 8, if there is a difference among surface temperatures at a referring point PB1, a reference point PA, and a referring point PB2 arranged in positions in the longitudinal direction of the steel sheet, this means that heat transfer in the longitudinal direction has occurred inside the steel sheet. On the other hand, if there is a difference in among surface temperatures at a referring point PB3, the reference point PA, and a referring point PB4 arranged in positions in the width direction of the steel sheet, this means heat transfer in the width direction has occurred inside the steel sheet. Therefore, information concerning a direction of the heat transfer inside the steel sheet can be reflected based on the surface temperature information at the reference point and these referring points. From such a reason, in the present embodiment, not only a mode in which actual measurement data of a surface temperature at a reference point and actual measurement data of a surface temperature at a referring point set based on the reference point are used as a surface temperature information data set but also the difference between the actual measurement data of a surface temperature at the reference point and the reference point of the surface temperature at the referring point set based on the reference point may be used to configure the surface temperature information data set. This is because the difference between the surface temperatures at the reference point and the referring point is also information concerning the heat transfer inside the steel sheet.

By acquiring the surface temperature information at the reference point and these referring points in a plurality of positions in the cooling process of the steel sheet, a temporal change about the behavior of the heat transfer inside the steel sheet can also be reflected in the material quality prediction model and the prediction accuracy by the material quality prediction model is improved. The surface temperature information at the reference point and these referring points is preferably acquired in two to ten positions from the start to the end of the cooling of the steel sheet S. This is because the information concerning the temporal change about the behavior of the heat transfer on the inside of the steel sheet S can be acquired by acquiring surface temperature information in two or more positions and, even if surface temperature information is acquired in more than ten positions, prediction accuracy by the material quality prediction model is not improved so much.

### [Material quality prediction unit]

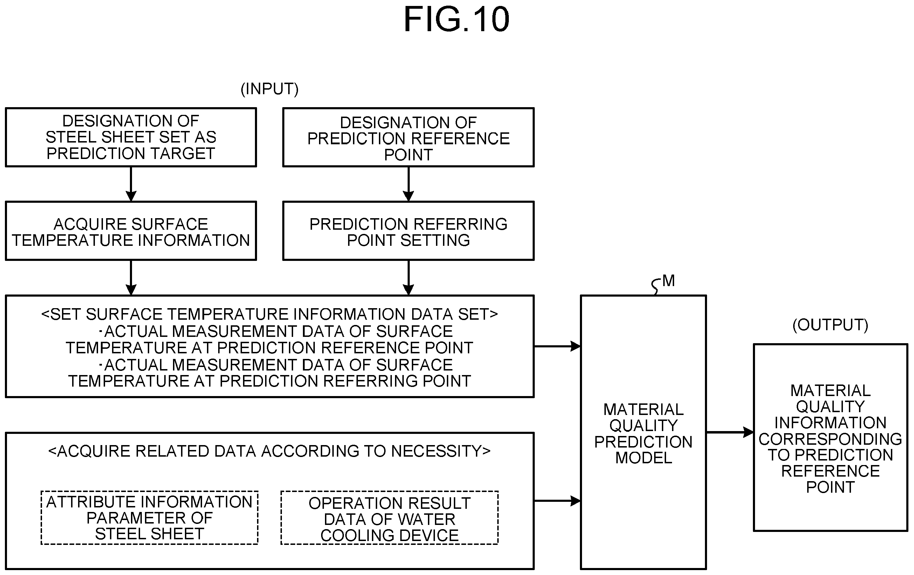

A steel sheet material quality prediction unit according to the embodiment of the present invention may be installed in the control computer 10 or the host computer 11 illustrated in FIG. 2. The material quality prediction unit may be installed in a control computer for a refining process for controlling the processing of the refining process in the refining equipment illustrated in FIG. 1. The material quality prediction unit may be installed as a part of these computers or may be configured as hardware separate from these computers. The material quality prediction unit may be installed in a tablet terminal including a communication function with the computer. However, it is assumed that the material quality prediction unit includes means capable of communicating with the control computer 10 or the host computer 11 illustrated in FIG. 2 and can acquire the surface temperature information of the steel sheet S acquired in the cooling equipment 3. An operation of the steel sheet material quality prediction unit according to the embodiment of the present invention is explained below with reference to FIG. 10.

FIG. 10 is a diagram for explaining the operation of the steel sheet material quality prediction unit according to the embodiment of the present invention. The operation illustrated in FIG. 10 is started at timing when the material quality prediction unit has acquired, for a steel sheet having passed through the cooling equipment 3, from the control computer 10 or the host computer 11, information for specifying the steel sheet such as a product number and a manufacturing number of a steel sheet set as a target of the material quality prediction and the material quality prediction unit has acquired information concerning a prediction reference point as a position where material quality should be predicted in the plane of the steel sheet. Note that the "prediction reference point" used in the material quality prediction unit is different from the reference point used when the result data is accumulated in the database unit 20a of the material quality prediction model generation unit 20 and any position of the steel sheet set as a target of the material quality prediction can be designated.

Subsequently, based on information for specifying a target steel sheet, the material quality prediction unit acquires surface temperature information collected when the steel sheet passed through the cooling equipment 3 and stored in the control computer 10 or the host computer 11. The surface temperature information of the steel sheet is result data of surface temperature associated with position information in the plane of the steel sheet. On the other hand, when the prediction reference point is designated, the material quality prediction unit sets a prediction referring point based on the prediction reference point. The prediction referring point used in the material quality prediction unit is set for the prediction reference point in the same positional relation as the positional relation of the referring point used to accumulate the result data in the database unit 20a of the material quality prediction model generation unit 20. That is, the material quality prediction unit applies referring points that are as many as the referring points corresponding to the reference points accumulated in the database unit 20a of the material quality prediction model generation unit 20 and are in the same positional relation in the distance and the direction from the reference point.

The material quality prediction unit acquires actual measurement data of the surface temperature of the steel sheet corresponding to the positions of the prediction reference point and the prediction referring point set in this way from the surface temperature information of the steel sheet based on the position information of the prediction reference point and the prediction referring point. Consequently, a surface temperature information data set as input to the material quality prediction model M is configured. Note that, since the surface temperature information of the steel sheet is discrete information associated with a coordinate in the plane of the steel sheet, in the present embodiment, the distance between the prediction reference point and the prediction referring point explained above is at least twice larger compared with a section (a collection pitch of the surface temperature information) concerning the position in the plane of the steel sheet from which the surface temperature information of the steel sheet is acquired. On the other hand, when an attribute information parameter of the steel sheet is used as input to the material quality prediction model M or when the operating parameter of the water cooling device 4 is used, each result data is sent from the host computer 11 or the storage device of the control computer 10 connected to the host computer 11 to the material quality prediction unit.

Through the above processing, the material quality prediction unit inputs the surface temperature information data set to the material quality prediction model M as input data to thereby output the material quality information of the steel sheet after having passed through the cooling equipment in the position corresponding to the prediction reference point set on the steel sheet.

On the other hand, the material quality prediction unit can obtain a material quality prediction result of the entire width and the entire length of the steel sheet by outputting the material quality information corresponding to the prediction reference points while changing the prediction reference point used for the material quality prediction in the plane of the steel sheet. It is possible to determine acceptance or rejection of the material quality of the steel sheet based on the material quality prediction result of the entire width and the entire length of the steel sheet obtained in this way. Specifically, it is possible to add a treatment process for determining whether a predetermined material quality standard is satisfied for the entire plane of the steel sheet, cutting a portion not satisfying the material quality standard, or allocating the portion to a product having specifications different from the original plan. The treatment process refers to an additional process different from the original production plan. Consequently, it is possible to prevent a product in which the material quality of the steel sheet as a product is non-uniform from being shipped and it is possible to provide a steel sheet in which the material quality in the plane is uniform.

### <Example 1>

In this example, in the offline type heat treatment equipment 1 illustrated in FIG. 1, a steel sheet in a room temperature state from which a scale of a surface was removed by performing shot blasting in preliminary treatment equipment in advance was used. Thereafter, the steel sheet was heated to 930°C in the heating furnace 2 in a nitrogen atmosphere, thereafter cooled in the cooling equipment 3, and cooled at a target temperature of 430°C set as a cooling stop temperature to produce tempered steel. The cooling equipment 3 is disposed on the downstream side of the heating furnace 2. Seven pairs of water cooling nozzles 41a and 41b and eight pairs of restraint rolls 42a and 42b configuring the water cooling device 4 are disposed on the inside of the cooling equipment 3. Flat spray nozzles were used as the water cooling nozzles 41a and 41b.

In the present example, a scanning type surface thermometer capable of measuring the temperature in the width direction of the steel sheet was installed at the inlet portion of the cooling equipment 3 2.0 m away from the heating furnace 2 and a scanning type surface thermometer was also installed in a position 3.0 m away from the outlet portion of the water cooling device 4. Consequently, a cooling start temperature was measured over the entire width and the entire length of the steel sheet in the inlet portion of the cooling equipment 3 and a cooling stop temperature was measured over the entire width and the entire length of the steel sheet in the outlet portion of the cooling equipment 3. The cooling start temperature and the cooling stop temperature were stored as surface temperature information of the steel sheet in the storage device of the host computer 11 via the control computer 10. Note that the cooling start temperature and the cooling stop temperature of the steel sheet measured in this example were 910°C ± 10°C and 450°C ± 50°C in the in-plane center of the steel sheet.

In this example, a thick steel sheet having sheet thickness of 12 mm and tensile strength of 780 MPa class (standard: yield stress of 685 MPa or more and tensile strength of 780 to 930 MPa) was used as a steel sheet set as a target of material quality prediction. In generating the material quality prediction model, learning data was acquired using one hundred thick steel sheets classified into the same category in size and standard. For the thick steel sheets, one to five reference points selected from the positions in the steel sheet plane such as the leading end portion, the tail end portion, the width direction end portion, and the central portion of the steel sheet were set. Two referring points based on the set reference points were set in each of the longitudinal direction and the width direction of the steel sheet and the distance between the reference points and the referring points was set to 50 mm. Consequently, a set of surface temperature information data sets having four referring points for one reference point and configured by five pieces of surface temperature information acquired by one surface temperature measurement device was generated. This means that a set of surface temperature information data sets configured by surface temperature information of 10 points for one reference point was generated through the cooling process by the cooling equipment 3.

On the other hand, as attribute information parameters selected from the attribute information of the steel sheet, the component composition, the sheet width, and the sheet length of the steel sheet were selected and, as for the component compositions of the steel sheet, the C content, the Si content, the Mn content, the Cr content, and the Mo content of the steel sheet were included in the attribute information parameters of the steel sheet as % by weight. These pieces of information were stored in the host computer 11. Note that the steel sheet included P, Ti, S, Al, and N besides these alloy components. However, within the above category, there was no significant influence on prediction accuracy of a steel sheet material quality prediction model explained below. Therefore, these alloy components were not included in the attribute information parameters of this example. Further, in this example, as the operation result data of the water cooling device 4, a total jetted water amount of the water cooling device 4, the number of jetting zones, and conveying speed of the steel sheet in the cooling equipment 3 were selected and included in the operation result data. Further, as the material quality information of the steel sheet used in this example after having passed through the steel sheet cooling equipment 3, the tensile strength and the yield stress obtained by the tensile test using the test piece sample collected from the position corresponding to the reference point in the refining process were used.

In this example, heat treatment for the one hundred steel sheets was performed and result data of material quality information in the positions corresponding to the respective reference points, result data of the surface temperature information data set for the reference points, the attribute information parameters explained above, and the operation result data of the water cooling device 4 explained above were accumulated in the database unit 20a. The material quality prediction model M of the steel sheet after having passed through the cooling equipment 3 was generated by machine learning using these learning data. As an algorithm of machine learning, a neural network was used and intermediate layers of the neural network were three layers and the number of nodes was five for each of the intermediate layers. A sigmoid function was used as an activation function.

The material quality prediction model M generated in this way was sent to a tablet terminal communicable with the control computer in the refining process and was set as a material quality prediction model of a material quality prediction unit configured inside the tablet terminal. The material quality prediction unit in this example includes a function of outputting a material quality prediction result for the entire width and the entire length of the steel sheet by acquiring material quality information in a position corresponding to a prediction reference point while variously changing the prediction reference point set in the plane of the steel sheet. The predicted material quality information and the surface temperature information acquired in the cooling process can be displayed and output as an image on the tablet terminal.

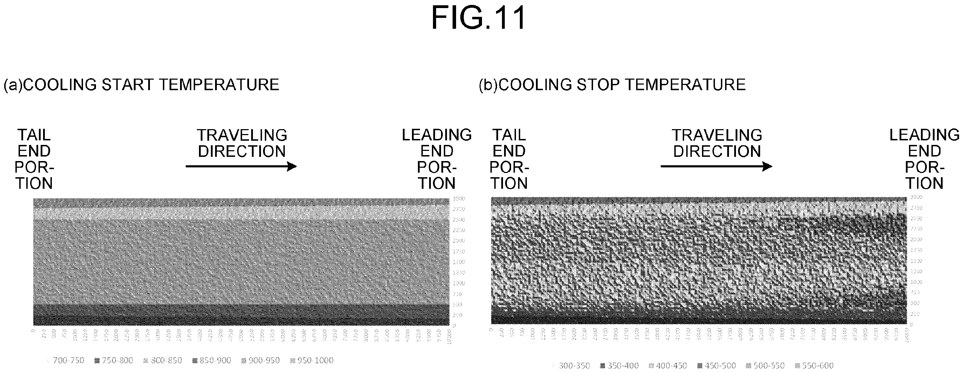

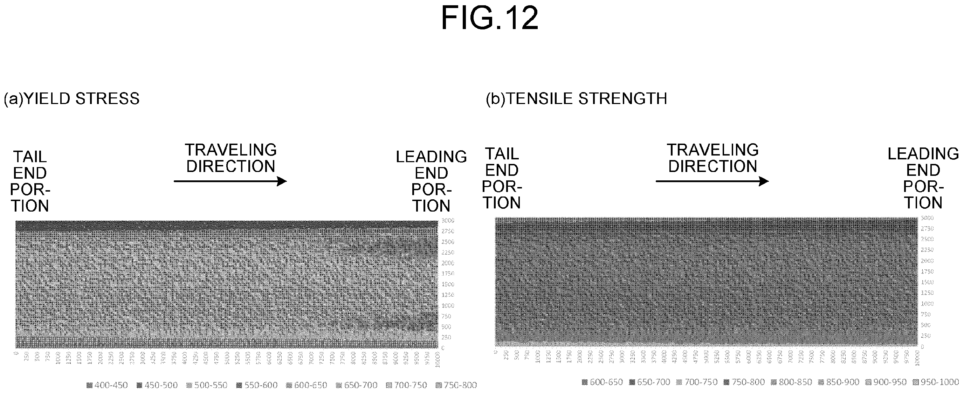

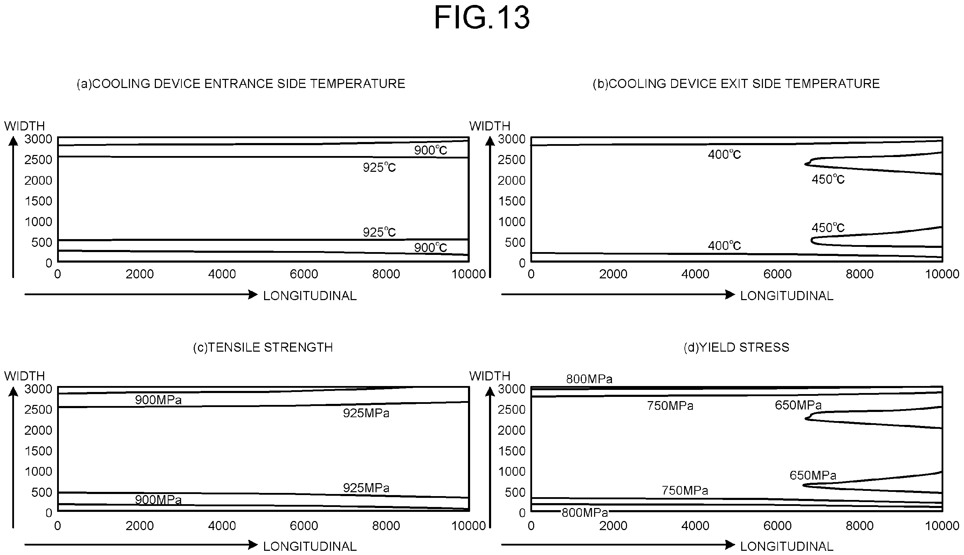

FIG. 11(a) illustrates an example of surface temperature information obtained by a surface temperature measurement device disposed in the inlet portion of the cooling equipment acquired in the cooling process. FIG. 11(b) illustrates an example of surface temperature information obtained by a surface temperature measurement device disposed in the outlet portion of the cooling equipment. FIG. 12(a) illustrates an example in which yield stress is displayed as material quality information according to this example. FIG. 12(b) illustrates an example in which tensile strength is displayed. In this way, the material quality information in the entire width and the entire length of the steel sheet can be predicted by the material quality prediction unit. The tablet terminal includes a function of displaying, with contour lines, the surface temperature information and the material quality information from such a color image and can display images illustrated in, for example, FIG. 13(a) to 13(d). By using such a material quality prediction result, it is possible to easily determine whether a region where the material quality does not satisfy predetermined specifications is present in the plane of the steel sheet.

In an example illustrated in FIG. 13(a), a state is seen in which a cooling start temperature on the surface of the steel sheet slightly drops at the width direction end portion of a thick steel sheet. This is considered to be because, although it is estimated that the thick steel sheet is almost uniformly heated in the heating furnace, heat radiation from the side surface of the thick steel sheet occurred while the thick steel sheet was extracted from the heating furnace and reached the surface temperature measurement device disposed at the inlet portion of the cooling equipment. In FIG. 13(b), it can be seen that the cooling stop temperature on the surface of the thick steel sheet drops at the width direction end portion of the thick steel sheet, the cooling stop temperature is slightly higher in the regions of 1/4 w and 3/4 w in the width direction of the thick steel sheet, and the cooling stop temperature at the leading end portion of the steel sheet is slightly higher than the cooling stop temperature at the rear end portion in the longitudinal direction of the thick steel sheet. The temperature drop at the end portion of the steel sheet is considered to have occurred because of the influence of the flow, fluctuation, and the like of the cooling water as characteristics of the water cooling device in addition to the supercooling that occurred on the upstream side of the water cooling device. Note that it is surmised that a temperature change in the longitudinal direction of the steel sheet is also caused by such characteristics of the water cooling device.

As for predicted values of the tensile strength and the yield stress illustrated in FIGS. 13(c) and 13(d), the tensile strength is lower and the yield stress is higher at the width direction end portion of the steel sheet. The yield stress is lower in the regions of 1/4 w and 3/4 w in the width direction of the steel sheet. Further, in the longitudinal direction of the steel sheet, the yield stress at the leading end portion is lower than the yield stress at the trailing end portion. Such a tendency is correlated with the surface temperature information acquired at the outlet portion of the cooling device illustrated in FIG. 13(b). The yield stress tends to increase as the cooling stop temperature is lower.

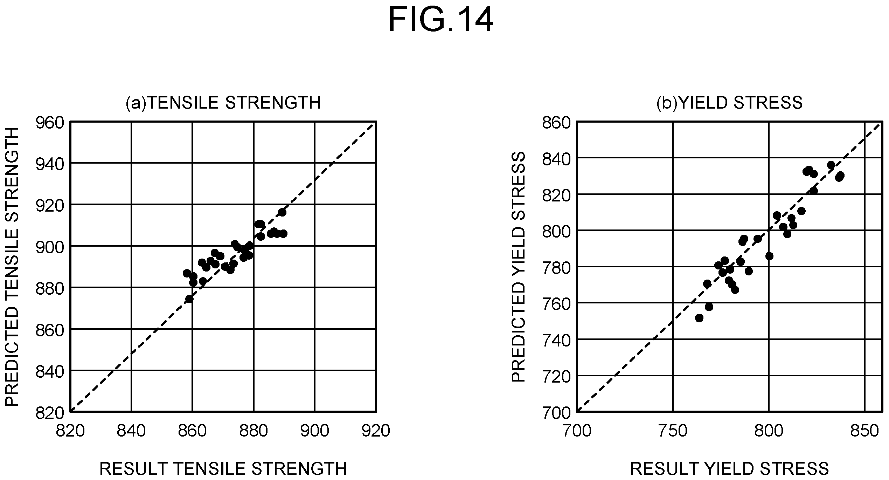

Subsequently, for the steel sheets illustrated in FIGS. 13(a) to 13(d), test piece samples were collected from optionally selected thirty parts including the vicinities of the leading end portion and the tail end portion in the plane and the vicinities of both the end portions in the width direction and a tensile test was performed. FIGS. 14(a) and 14(b) illustrate results obtained by comparing a result of the tensile test with material quality information illustrated in FIGS. 13(c) and 13(d). As illustrated in FIG. 14(a), concerning the tensile strength, a standard deviation of an error between a predicted value and an actual measurement value was 4.6 MPa. As illustrated in FIG. 14(b), concerning the yield stress, a standard deviation of an error between a predicted value and an actual measurement value was 9.1 MPa. Consequently, according to the present invention, it has been confirmed that practically sufficient prediction accuracy of the material quality can be obtained.

As explained above, according to this example, for example, at a stage of product shipment of the steel sheet, whereas quality assurance was conventionally performed based on material quality information of only a part where a mechanical test was performed, it is possible to guarantee the material quality over the entire width and the entire length. Further, if the material quality prediction model generated according to this example is used, for example, prior to the heat treatment process of the steel sheet, by adjusting the operating parameters of the water cooling device according to variation in the component composition of the steel sheet, it is possible to manufacture a steel sheet with less variation in material quality.

### <Example 2>

In the following explanation, a result of applying the steel sheet material quality prediction method according to the present embodiment to the case of outputting other material quality information is explained. In this example, a steel sheet excellent in wear resistance was manufactured using the offline type heat treatment equipment 1 illustrated in FIG. 1. As the steel sheet subjected to heat treatment using the offline type heat treatment equipment 1, a steel material containing, in terms of % by mass, a component composition of C: 0.12 to 0.50%, Si: 0.01 to 1.0%, Mn: 0.01 to 2.5%, P: 0.040% or less, S: 0,040% or less, Cr: 0.01 to 3.0%, Ti: 0.001 to 1.5%, B: 0.0001 to 0.010%, Al: 0.10% or less, and N: 0.050% or less and including the balance Fe and unavoidable impurities was used. The steel material was subjected to hot rolling in advance using a hot rolling line under conditions of heating temperature of 1150 to 1250°C and a cumulative rolling reduction rate of 90 to 97% in a temperature range of an Ar3 transformation point or higher to form a steel sheet having a sheet thickness of 12 to 13 mm and thereafter cooled to room temperature. In this example, the steel sheet after the hot rolling was reheated using the heating furnace 2 of the offline type heat treatment equipment 1 illustrated in FIG. 2 and the steel sheet was quenched using the cooling equipment 3. As heat treatment conditions for the steel sheet, a cooling start temperature was set to 930°C ± 10°C and operation conditions of the water cooling device 4 were set to set a target cooling stop temperature to 250°C ± 50°C.

In this example, under the above conditions, one hundred steel sheets were subjected to heat treatment under conditions in which the total amount of water to be jetted by the water cooling device 4, the number of jetting zones, and the conveying speed of the steel sheet in the cooling equipment 3 were changed. The following material quality information was selected as the material quality information of the steel sheet in this example.

·Brinel hardness HBW of the steel sheet surface obtained by a hardness test
- Absorbed energy (J) at -40°C acquired by a Charpy impact test
- Repeated yield strength (MPa) obtained by a repeated stress strain test
- Fatigue crack propagation speed (m/cycle) in a stress expansion coefficient range ΔK1 = 15 MPaVm acquired by a fatigue crack propagation test

The material quality information of the steel sheet is acquired from the steel sheet after having passed through the cooling equipment 3. A test piece sample for performing the above test was collected from a position corresponding to a reference point of the steel sheet conveyed to the refining equipment in the offline heat treatment process. The test piece sample collected according to one reference point was 200×200 mm and a test piece to be subjected to the above test was collected from the collected test piece sample. As the reference point set in the steel sheet, one to five reference points were individually set according to the steel sheet to be manufactured as five points of the leading end portion, the tail end portion, the width direction end portions (a work side and a drive side), and the in-plane central portion of the steel sheet. Two referring points corresponding to the set reference points were set in each of the longitudinal direction and the width direction of the steel sheet and all of the distances between the reference points and the referring points were set to 150 mm. Consequently, four referring points were associated with one reference point.

In this example, temperature measurement devices for measuring the surface temperature of the steel sheet were disposed in two positions, i.e., a position 1 m away from the inlet of the water cooling device 4 and a position 5 m away further to the downstream side than the water cooling device 4. A cooling start temperature and a cooling stop temperature of the steel sheet were measured by the temperature measurement devices. That is, in this example, since the five pieces of temperature information including the temperatures of referring points are acquired in two parts according to one reference point as a surface temperature information data set of the steel sheet, a surface temperature information data set having ten pieces of surface temperature information is acquired for the respective reference points.

The surface temperature information data set acquired for each of the reference points as explained above was accumulated in the database unit 20a as learning data together with information for specifying the steel sheet and information for specifying the reference points. In the database unit 20a, result data of the sheet width of the steel sheet is accumulated as an attribute information parameter of the steel sheet acquired for each of steel sheets and a C content, an Si content, an Mn content, and a Cr content were accumulated as result data concerning a component composition of the steel sheet. Note that, although the steel material used in this example also includes other component compositions, because of a combination of the component compositions with the operation parameters and the like of the water cooling device 4, the influence of the component compositions on prediction accuracy of a material quality prediction model was not large. Therefore, the material quality prediction model was generated by being limited to a main component composition of the steel material.

Further, in the database unit 20a, result data of the total jetted water amount and the number of jetting zones of the water cooling device 4 and the conveying speed of the steel sheet in the cooling equipment 3 were accumulated as the operation result data of the water cooling device 4. On the other hand, as material quality information acquired for each of reference points of the steel sheet, the test result obtained using the test piece collected from the test piece sample was accumulated in the database unit 20a. Note that, in the database unit 20a, the surface temperature information data set, the attribute information parameter of the steel sheet, the operation result data of the water cooling device 4, and the result data of the material quality information of the steel sheet were associated based on the information for identifying the steel sheet and the information for specifying the reference points.

Thereafter, in the machine learning unit 20b of the material quality prediction model generation unit 20, the material quality prediction model M of the steel sheet after having passed through the cooling equipment 3 was generated by machine learning performed using a plurality of learning data in which the surface temperature information data set, the attribute information parameter of the steel sheet, and the operation result data of the water cooling device 4 were set as input result data and the material quality information of the steel sheet corresponding to these input result data was set as output result data. A neural network was used as an algorithm of the machine learning and intermediate layers of the neural network were five layers and the number of nodes was eight for each of the intermediate layers. A sigmoid function was used as an activation function.

The steel sheet material quality prediction model M generated by the material quality prediction model generation unit 20 was sent to a tablet terminal communicable with the control computer of the refining equipment and set as a material quality prediction model of a material quality prediction unit configured on the inside of the tablet terminal. The control computer of the refining equipment includes the host computer 11 common to the control computer 10 of the heat treatment equipment and is configured to be capable of acquiring result data acquired by the control computer 10 of the heat treatment equipment and result data acquired by the surface temperature measurement device. Consequently, the material quality prediction unit can acquire actual measurement data of surface temperatures at a prediction reference point and a prediction referring point of the steel sheet acquired in the offline type heat treatment equipment 1, result data of the attribute information parameter of the steel sheet, and operation result data of the water cooling device 4 and can output material quality information of the steel sheet after having passed through the cooling equipment in a position corresponding to the prediction reference point of the steel sheet by inputting the actual measurement data of the surface temperature, the result data of the attribute information parameter of the steel sheet, and the operation result data of the water cooling device 4 to the material quality prediction model M.

In this example, six steel sheets that are the same as the steel sheet explained above were prepared as a steel sheet for a test and heat treatment was performed again using the offline type heat treatment equipment 1. At that time, after the steel sheet passed through the cooling equipment 3 of the offline type heat treatment equipment 1, the material quality prediction unit acquired surface temperature information of the entire surface of the steel sheet measured by the surface temperature measurement device. The material quality prediction unit outputted material quality information of the steel sheet in positions corresponding to prediction reference points while changing a prediction reference point of the steel sheet in the plane of the steel sheet. Consequently, a predicted value of the material quality information on the entire surface of the steel sheet was obtained from the material quality prediction unit.

From the steel sheet manufactured for the test, test piece samples were collected from the leading end portion, the tail end portion, and optionally selected three positions in the plane and the material quality information of the steel sheet was acquired by the same test as the test explained above. That is, result data of thirty pieces of material quality information in total was acquired from five positions of each of the six steel sheets. The material quality prediction unit outputted predicted values of material quality information of prediction reference points corresponding to the positions where the test piece samples were collected and compared the predicted values with result data of the material quality information. Errors between the predicted values and result values concerning the material quality information of the steel sheet were aggregated and a standard deviation σ thereof was calculated. Then, the following results were obtained.

·A standard deviation σ 5.2 HBW of a prediction error with respect to an average value 366 HBW of result data of Brinell hardness
- A standard deviation σ 2.3J of a prediction error with respect to an average value 22J of result data of absorbed energy
- A standard deviation σ 43.6 MPa of a prediction error with respect to an average value 1057 MPa of result data of repeated yield strength
- A standard deviation σ 0.75×10⁻⁹ m/cycle of a prediction error with respect to an average value 4.12×10⁻⁹ m/cycle of result data of fatigue crack propagation speed

From the above, it has been known that the steel sheet material quality prediction model M generated in this example can predict, with practically sufficient accuracy, the material quality information of the steel sheet measured by the hardness test, the Charpy impact test, the repeated stress-strain test, and the fatigue crack propagation test.

### <Example 3>

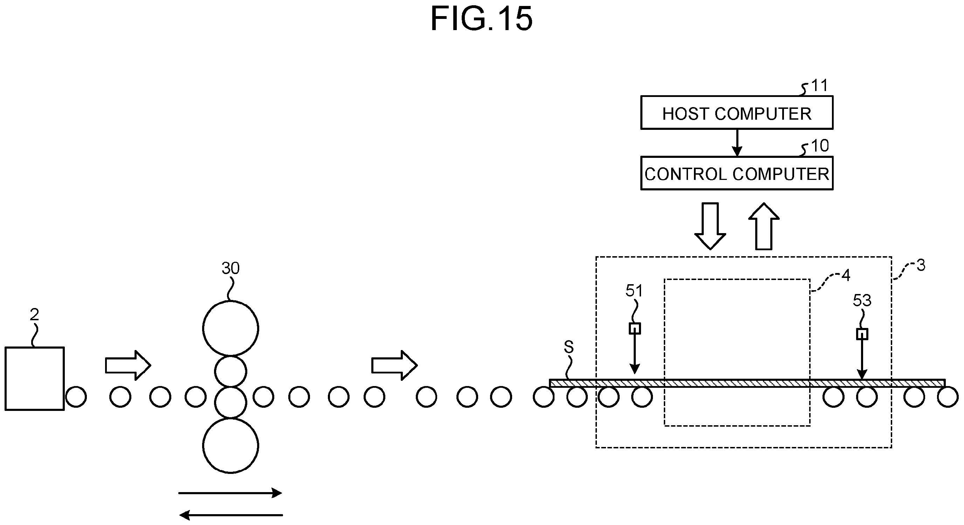

In the following explanation, a result of applying the steel sheet material quality prediction method according to the present embodiment to wear resistant steel having excellent bending workability is explained. In this example, quenching treatment was performed by the cooling equipment 3 of the hot rolling line illustrated in FIG. 15 for executing the online type heat treatment process.

The hot rolling line illustrated in FIG. 15 includes the heating furnace 2, a rolling mill 30, and the cooling equipment 3. In the heating furnace 2, a slab of a steel material is heated to a predetermined temperature. The rolling mill 30 is a reverse type rolling mill, and is equipment for performing multi-pass rolling on a steel sheet so that the steel plate has predetermined sheet thickness and predetermined plate width. The steel sheet rolled into predetermined dimensions by the rolling mill 30 is heated to a high temperature state and thereafter subjected to a heat treatment process using the cooling equipment 3. Temperature measurement devices 51 and 53 are disposed on the upstream side and the downstream side of the cooling equipment 3. As the cooling equipment 3 disposed in the online type heat treatment equipment, the same cooling equipment as the cooling equipment 3 illustrated in FIG. 2 is used.

In this example, the tempering treatment for the steel sheet was performed after the online type heat treatment process using the cooling equipment 3 of the hot rolling line was executed. In the tempering treatment, cooling of the steel sheet using the cooling equipment was not performed and material quality information was acquired from the steel sheet after the tempering treatment. The steel sheet subjected to the tempering treatment by the above manufacturing process is a steel material containing, in terms of % by mass, a component composition of C: 0.06 to 0.25%, Si: 0.01 to 0.8%, Mn: 0.5 to 2%, P: 0.010% or less, S: 0,003% or less, Al: 0.005 to 0.1%, N: 0.0005 to 0.008%, and Mo: 0.01 to 1% and including the balance Fe and unavoidable impurities. The steel material was hot-rolled using the hot rolling line under conditions of a heating temperature of 1100°C and an un-recrystallized area reduction rate of 40 to 50% to form a steel sheet having sheet thickness of 10 mm and thereafter heat-treated with cooling equipment having the same configuration as the configuration of the cooling equipment 3 illustrated in FIG. 2 under conditions of a cooling start temperature of 750 to 780°C, a target cooling stop temperature of 200 to 250°C, and a target value 65 to 70°C/sec of average cooling speed at 500 to 700°C. Thereafter, the steel sheet was reheated at 580 to 600°C to perform the tempering treatment.

In this example, in the online type heat treatment process, the quenching treatment was executed on one hundred steel sheets under the conditions in which the total amount of jetted water and the number of jetting zones of the water cooling device 4 and the conveying speed of the steel sheet in the cooling equipment 3 were changed. Thereafter, after the steel sheet was tempered under the same tempering conditions, air cooling was performed on the cooling bed, a test piece sample was collected in the refining equipment, and material quality information of the steel sheet was acquired from the following test.

·Tensile strength (MPa) and yield stress (MPa) obtained by a tensile test
·Critical bending radius acquired by a bending test (a ratio of a bending radius to sheet thickness of the steel sheet)

The material quality information of the steel sheet is acquired from the steel sheet having passed through the cooling equipment 3 of the hot rolling line and subjected to the quenching treatment and thereafter subjected to the tempering treatment by reheating. A test piece sample for performing the above test was collected from a position corresponding to a reference point of the steel sheet conveyed to the refining equipment after the tempering treatment. A test piece sample collected according to one reference point was 150×150 mm and a test piece to be subjected to the above test was collected from the collected test piece sample. As the reference point set in the steel sheet, one to five reference points were individually set according to the steel sheet to be manufactured as five points of the leading end portion, the tail end portion, the width direction end portions (a work side and a drive side), and the in-plane central portion of the steel sheet. Two referring points corresponding to the set reference points were set in the width direction of the steel sheet and the distance between the reference points and the referring points was set to 80 mm. Consequently, two referring points were associated with one reference point.

Temperature measurement devices for measuring the surface temperature of the steel sheet were disposed in two positions, that is, a position 2 m away from the inlet of the water cooling device 4 disposed on the downstream side of the hot rolling line and a position 5 m away further to the downstream side than the water cooling device 4. A cooling start temperature and a cooling stop temperature of the steel sheet were measured by the temperature measurement devices. That is, in this example, three pieces of temperature information including the temperature of the referring point are acquired in two places according to one reference point as a surface temperature information data set of the steel sheet. Therefore, a surface temperature information data set having six pieces of surface temperature information is acquired for the respective reference points.

The surface temperature information data set acquired for each of the reference points as explained above was accumulated in the database unit 20a as learning data together with information for specifying the steel sheet and information for specifying the reference points. In the database unit 20a, the result data of the sheet width and the length of the steel sheet as the attribute information parameter of the steel sheet acquired for each of steel sheets and the C content, the Si content, the Mn content, and the Mo content are accumulated as the result data concerning the component composition of the steel sheet. Note that, although the steel material used in this example also includes other component compositions, the influence of the component compositions on prediction accuracy of the material quality prediction model was not conspicuous because of the combination with the operation parameters and the like of the water cooling device 4. Therefore, the material quality prediction model was generated by being limited to the main component compositions of the steel material. Further, in the database unit 20a, result data of the total jetted water amount and the number of jetting zones of the water cooling device 4 and the conveying speed of the steel sheet in the cooling equipment 3 were accumulated as the operation result data of the water cooling device 4. On the other hand, as the material quality information acquired for each of the reference points of the steel sheet, the test result obtained using the test piece collected from the test piece sample was accumulated in the database unit 20a. Note that, in the database unit 20a, the surface temperature information data set, the attribute information parameter of the steel sheet, the operation result data of the water cooling device 4, and the result data of the material quality information of the steel sheet were associated based on the information for identifying the steel sheet and the information for specifying the reference points.

Thereafter, in the machine learning unit 20b of the material quality prediction model generation unit 20, the material quality prediction model M of the steel sheet after having passed through the cooling equipment 3 of the hot rolling line and subjected to the tempering treatment was generated by machine learning performed using a plurality of learning data in which the surface temperature information data set, the attribute information parameter of the steel sheet, and the operation result data of the water cooling device 4 were set as input result data and the material quality information of the steel sheet corresponding to these input result data was set as output result data. A neural network was used as an algorithm of the machine learning and intermediate layers of the neural network were four layers and the number of nodes was six for each of the intermediate layers. A sigmoid function was used as an activation function. Note that operating conditions of the tempering treatment executed on the steel sheet having passed through the cooling equipment 3 are set in advance according to a standard and a steel type of the steel sheet. Variation in a tempering temperature in reheating is small. Therefore, a degree of influence on the material quality information of the steel sheet is relatively smaller compared with the operation parameters in the cooling process for the steel sheet in the cooling equipment 3. It is possible to generate a highly accurate material quality information model of the steel sheet even if the operation parameters of the tempering process are not used as input to the material quality prediction model.

The steel sheet material quality prediction model M generated by the material quality prediction model generation unit 20 was sent to a tablet terminal communicable with the control computer of the refining equipment and set as a material quality prediction model of a material quality prediction unit configured on the inside of the tablet terminal. The control computer of the refining equipment includes the host computer 11 common to the control computer 10 of the hot rolling line and is configured to be capable of acquiring result data acquired by the control computer 10 of the hot rolling line and result data acquired by the surface temperature measurement device. Consequently, the material quality prediction unit can acquire actual measurement data of surface temperatures at a prediction reference point and a prediction referring point of the steel sheet acquired in the cooling equipment of the hot rolling line, result data of the attribute information parameter of the steel sheet S, and operation result data of the water cooling device 4 and can output material quality information of the steel sheet after having passed through the cooling equipment in a position corresponding to the prediction reference point of the steel sheet by inputting the actual measurement data of the surface temperature, the result data of the attribute information parameter of the steel sheet S, and the operation result data of the water cooling device 4 to the material quality prediction model M.

In this example, ten steel sheets that are the same as the steel sheet explained above were prepared as a steel sheet for a test and the quenching treatment using the cooling equipment of the hot rolling line and the tempering treatment by the reheating were performed again. At that time, after the steel sheet passed through the cooling equipment 3 of the hot rolling line, the material quality prediction unit acquired surface temperature information of the entire surface of the steel sheet measured by the surface temperature measurement device. The material quality prediction unit outputted material quality information of the steel sheet in positions corresponding to prediction reference points while changing a prediction reference point of the steel sheet in the plane of the steel sheet. Consequently, a predicted value of the material quality information on the entire surface of the steel sheet was obtained from the material quality prediction unit. From the steel sheet manufactured for the test, test piece samples were collected from optionally selected three parts in the steel sheet plane and the material quality information of the steel sheet was acquired by the same test as the test explained above. That is, result data of thirty pieces of material quality information in total was acquired from three positions of each of the ten steel sheets. The material quality prediction unit outputted predicted values of material quality information of prediction reference points corresponding to the positions where the test piece samples were collected and compared the predicted values with result data of the material quality information. Errors between the predicted values and result values concerning the material quality information of the steel sheet were aggregated and a standard deviation σ thereof was calculated. Then, the following results were obtained.

·A standard deviation σ 12.3 MPa of a prediction error with respect to an average value 1005 MPa of result data of tensile strength
·A standard deviation σ 9.2 MPa of a prediction error with respect to an average value 970 MPa of result data of yield stress
·A standard deviation 0.34 of a prediction error with respect to an average value 2.5 of result data of a limit bending radius (a ratio to sheet thickness)

From the above, it has been known that the steel sheet material quality prediction model M generated in this example can predict, with practically sufficient accuracy, the material quality information of the steel sheet measured by the tensile test and the bending test.

Although the embodiment to which the invention made by the present inventors is applied is explained above, the present invention is not limited by the description and the drawings forming a part of the disclosure of the present invention according to the present embodiment. That is, all of other embodiments, examples, operation techniques, and the like made by those skilled in the art based on the present embodiment are included in the scope of the present invention.

### Industrial Applicability

According to the present invention, it is possible to provide a method for generating a steel sheet material quality prediction model capable of generating a steel sheet material quality prediction model for accurately predicting material quality information of the steel sheet after having passed through cooling equipment. According to the present invention, it is possible to provide a steel sheet material quality prediction method capable of accurately predicting material quality information of the steel sheet after having passed through cooling equipment. Further, according to the present invention, it is possible to provide a steel sheet manufacturing method and manufacturing equipment capable of manufacturing a steel sheet excellent in material quality uniformity.

### Reference Signs List

1 OFFLINE TYPE HEAT TREATMENT EQUIPMENT
2 HEATING FURNACE
3 COOLING EQUIPMENT
4 WATER COOLING DEVICE
5, 51, 52, 53 TEMPERATURE MEASUREMENT DEVICE
6 TABLE ROLL
7 DRAINAGE PURGE NOZZLE
7a DRAINAGE PURGE
10 CONTROL COMPUTER
10a WATER COOLING CONDITION CALCULATION UNIT
11 HOST COMPUTER
20 MATERIAL QUALITY PREDICTION MODEL GENERATION UNIT
20a DATABASE UNIT
20b MACHINE LEARNING UNIT
30 ROLLING MILL
41a, 41b WATER COOLING NOZZLE
42a, 42b RESTRAINT ROLL
43 WATER COOLING FLOW RATE CONTROL DEVICE
54 SURFACE TEMPERATURE INFORMATION GENERATION DEVICE
M MATERIAL QUALITY PREDICTION MODEL
PA REFERENCE POINT
PB1, PB2, PB3, PB4 REFERRING POINT
5 STEEL SHEET
W COOLING WATER

## Claims

1. A method for generating a steel sheet material quality prediction model in steel sheet cooling equipment, the cooling equipment including a water cooling device that cools a steel sheet by jetting cooling water to a heated steel sheet and a temperature measurement device that measures a surface temperature of the steel sheet in a cooling process, the method comprising
a step of generating a material quality prediction model of the steel sheet after having passed through the cooling equipment with machine learning using a plurality of learning data in which
a surface temperature information data set including: actual measurement data of a surface temperature at a reference point set in advance on the steel sheet; and actual measurement data of a surface temperature at a referring point set based on the reference point, is set as input result data and
material quality information of the steel sheet after having passed through the cooling equipment in a position corresponding to the reference point on the steel sheet corresponding to the input result data is set as output result data.

2. The method for generating a steel sheet material quality prediction model according to claim 1, wherein at least a singularity of the referring point is set in a position in a longitudinal direction of the steel sheet and at least a singularity of the referring point is set in a position in a width direction of the steel sheet with respect to the reference point.

3. The method for generating a steel sheet material quality prediction model according to claim 1 or 2, wherein the material quality prediction model includes, as the input result data, an attribute information parameter selected from attribute information of the steel sheet.

4. The method for generating a steel sheet material quality prediction model according to any one of claims 1 to 3, wherein the material quality prediction model includes, as the input result data, at least one operation result data selected from operation result data of the water cooling device.

5. The method for generating a steel sheet material quality prediction model according to any one of claims 1 to 4, wherein machine learning selected from a neural network, decision tree learning, random forest, and support vector regression is used as the machine learning.

6. A steel sheet material quality prediction method in steel sheet cooling equipment, the cooling equipment including a water cooling device that cools the steel sheet by jetting cooling water to a heated steel sheet and a temperature measurement device that measures a surface temperature of the steel sheet in a cooling process, the method comprising
a step of predicting material quality information of the steel sheet after having passed through the cooling equipment by using a material quality prediction model generated by machine learning in which
a surface temperature information data set including: a surface temperature at a prediction reference point set in advance on the steel sheet; and a surface temperature at a prediction referring point set based on the prediction reference point, is set as input data and
material quality information of the steel sheet after having passed through the cooling equipment in a position corresponding to the prediction reference point on the steel sheet is set as output data.

7. A steel sheet manufacturing method, comprising a step of performing pass/fail determination of material quality of a steel sheet after having passed through the cooling equipment using the steel sheet material quality prediction method according to claim 6.

8. Steel sheet manufacturing equipment comprising:
steel sheet cooling equipment including a water cooling device that cools the steel sheet by jetting cooling water to a heated steel sheet and a temperature measurement device that measures a surface temperature of the steel sheet in a cooling process; and
a material quality prediction unit that outputs material quality information of the steel sheet after having passed through the cooling equipment, wherein
the material quality prediction unit outputs material quality information of the steel sheet using a machine learning model in which
a surface temperature information data set including: a surface temperature at a prediction reference point set in advance on the steel sheet; and a surface temperature at a prediction referring point set based on the prediction reference point, is set as input data and
material quality information of the steel sheet after having passed through the cooling equipment in a position corresponding to the prediction reference point on the steel sheet is set as output data.
